# EUROPEAN PATENT APPLICATION

(11) **EP 1 057 858 A2**
(43) Date of publication of application: **06.12.2000**
(21) Application number: 00401540.0
(22) Date of filing: 31.05.2000
(51) Int. Cl.: C08K 5/10

(54) **A citrate compound, a plasticizer, and a thermoplastic resin composition**

(30) Priority: 31.05.1999 JP 15295899; 01.11.1999 JP 31099399; 01.11.1999 JP 31105999; 28.01.2000 JP 2000020655; 25.02.2000 JP 2000049362
(71) Applicant: Daicel Chemical Industries, Ltd., Sakai-shi, Osaka-fu (JP); DAIHACHI CHEMICAL INDUSTRY CO., LTD., Osaka-shi, Osaka 541-0046 (JP)
(72) Inventor: Nakata, Koji, Himeji-shi, Hyogo-ken 671-1234 (JP); Ohnishi, Yasuhiro, Souraku-gun, Kyoto-fu 619-0237 (JP)
(74) Representative: Portal, Gérard

(57) **Abstract**

In the first aspect of the invention, there are provided a citrate compound represented by general formula [1] described below, 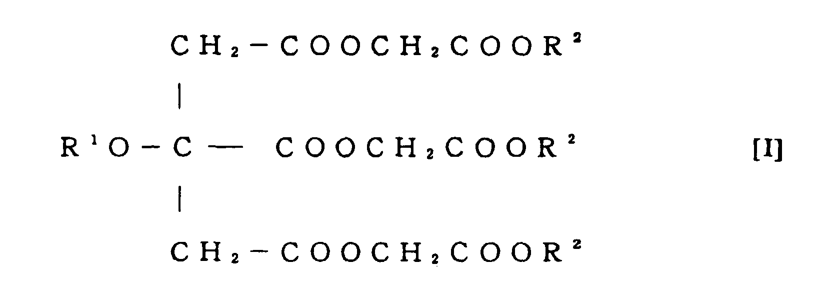 <in the formula, R¹ is a hydrogen atom or an aliphatic acyl group, and R² is an alkyl group>, and a plasticizer for thermoplastic resins which comprises the citrate compound which is not apt to bleed out so much.

In the second aspect of the invention, there are provided a thermoplastic resin composition which comprises a citrate compound and a thermoplastic resin such as a cellulose acetate-based resin, a styrene-based resin, and a polyvinyl chloride-based resin, and a cellulose acetate-based resin-made card having biodegradability which comprises a plasticizer which is not apt to bleed out of a cellulose ester-based resin and a cellulose ester-based resin.

## Description

### FIELD OF THE INVENTION

A first aspect of the invention relates to a novel citrate compound and plasticizer for thermoplastic resin. In more detail, it relates to a novel citrate compound which provides a resin composition.

By the use of the plasticizer for thermoplastic resin which is the citrate compound, the resin composition can be given an excellent compatibility, and in which the plasticizer does not bleed out scarcely, and volatility is low during molding.

A second aspect of the invention further relates to a thermoplastic resin composition in which a specified citrate compound is added to a thermoplastic resin. In more detail, it relates to a thermoplastic resin composition in which there is added a plasticizer which comprises a citrate compound having a specified chemical structure, which has an excellent workability in kneading, and which can maintain a variety of physical properties such as mechanical and thermal properties in a well-balanced state and, moreover, which does not cause problematic bleeding of the plasticizer involved in a variety of all resins. Still further, it relates to a biodegradable card which is composed of the thermoplastic resin composition which is a cellulose acetate-based resin composition. The card can be widely utilized as a card having thick thickness such as a credit card and cash card, and a card having thin thickness such as a prepaid card and a telephone card.

### BACKGROUND OF THE INVENTION

A cellulose acetate-based resin such as a cellulose acetate, a cellulose acetate propionate, and a cellulose acetate butylate has a characteristics such as toughness, and which is excellent in glossiness, transparency, oil resistance, and weatherability. However, since those usually cause discoloration and decomposition when it is thermally-melted without addition of plasticizers or solvents, an excellent molded article cannot be obtained. Further, also in a so-called precast method in which a sheet is obtained by dissolving, kneading, compressing, and molding using a variety of solvents, it is usually difficult to obtain a resin material for molding having an excellent ductility without the addition of plasticizers.

For that reason, a variety of plasticizers have been conventionally employed and there are typically exemplified phthalates, that is, dimethyl phthalate, diethyl phthalate, dibutyl phthalate, butylbenzyl phthalate, ethylphthalyl ethylene glycolate, etc., trimeritates, that is, trimethyl trimeritate, triethyl trimeritate, and tri-2-ethylhexyl trimeritate, etc., aliphatic dibasic acid esters, that is, di-2-ethylhexyl azelate, etc., phosphates, that is, tributyl phosphate, tributoxyethyl phosphate, triphenyl phosphate, tricresyl phosphate, cresyldiphenyl phosphate, and 2-ethylhexyldiphenyl phosphate, etc., and licinoleic acid esters, that is, methylacetyl licinolate, etc.

Further, in JP-B-86014168 Official Gazette, there is disclosed a cellulose acetate-based resin composition in which there is employed a polycaprolactone polyol having a specified chemical structure as a plasticizer.

The cellulose acetate-based resin composition containing a plasticizer is widely employed as, for example, sheets, films, pipes, rods, printing materials, decorations, frames for glasses, a handle for tools, a handle for eating tools, toys, and miscellaneous goods, etc. because of becoming easy in molding by decline of softening temperature. However, since the cellulose acetate-based resin is usually strong in polarity, it is required that there is selected a compound having a strong polarity as a plasticizer, and it has been desired that there is obtained a molded article which satisfies a variety of properties such as a high transparency, a non-volatilization property, and a non-migration property of a plasticizer by obtaining a composition having compatibility between both and a plasticizer effect by a plasticizer, and then using the composition.

In order to satisfy the requests, there is disclosed a thermoplastic composition containing a plasticizer such as a cellulose acetate-butylate, a cellulose acetate, tributyl citrate, triethyl citrate, and polyethylene glycol in US Patent 4731122 Specification. However, even in the composition by the combination thereof, compatibility between both is still insufficient and, in the case that a cellulose acetate having a low substitution degree is applied, since the plasticizer is diminished by evaporation or bleeding out during molding or after molding, there are caused a surface stain of a molded article, a decline of dimensional stability (an evaluation is conducted by volume retention ratio, hereinafter, the same) in a molded article, a change for worse in a working circumstance such as volatilization of a plasticizer during molding, it is the existing circumstances that there is not sufficiently satisfied the requests as a material for molding of cellulose acetate resins.

As a citric acid derivative which is employed as a plasticizer, there are known, in addition to the above-described ester compounds, many compounds which include an O-acylated compound such as an O-acetylated compound, a trimethylester, and a triethylester, etc. Further, acetyl triethyl citrate is known as a plasticizer for an ethylcellulose, and acetyl tributyl citrate is known as a plasticizer for plastics for food wrapping.

In the meantime, although a biodegradable card is already known, there are employed materials such as paper which are poor in water resistance and physical properties (tear resistance) as a base material thereof and, even though a biodegradable plastics, almost of those are insufficient in the physical properties compared to general-purpose plastics because of attaching importance to biodegradability.

Further, in the biodegradable plastics, there is also a resin to which a plasticizer is added, and there is a problem that the plasticizer added oozes out (bleeding) of surface of such the resin with a lapse of time. There is caused a problem that printing on a base material becomes occasionally difficult by the bleeding of the plasticizer, or a printing layer occasionally blisters or peels by the bleeding of the plasticizer after printing.

For that reason, there is desired a card in which although a resin shows physical properties which are not poor compared to those of the general-purpose plastics during use, after use, it is decomposed in natural circumstances and changed to valuable products and, in which a base material for the card is excellent in printing applicability. The purpose of the present invention is to provide a card in which a plasticizer is not apt to be bled out, and which is excellent in printing applicability and has same physical properties as the general-purpose plastics, and which is decomposed during dumping after use.

A polyvinylchloride homopolymer and a polyvinylchloridebased copolymer (hereinafter, there are merely named generically vinylchloride-based resin) containing a small amount of vinyl acetate and/or vinylidene chloride, etc. which are a copolymerizable component are excellent in mechanical properties, flame retardancy, weatherability, and electric properties (electric insulation properties, etc.) and other properties, and molded articles therefrom have been widely employed. The vinylchloride-based resin is poorer in the thermoplasticity at a large amount of vinylchloride components, and it is usually supplied as a vinylchloride-based resin composition in which a plasticizer is added.

As the plasticizer conventionally employed, there are dibutyl phthalate (DBP) and dioctyl phthalate (DOP) which are a phthalic acid-based plasticizer.

As a phosphoric acid-based plasticizer, there is tricresyl phosphate (TCP), etc. In kneading the vinylchloride-based resin with the plasticizers, working circumstances, for example, torque, odor, smoking, and a migration property (a bleeding property) are not sufficient and, there has been desired a development of a new plasticizer which is preferred for the vinylchloride-based resin.

In addition to the above-described cellulose acetate resin and vinylchloride resin, since plastics are generally light-weight, highly-strong, and excellent in solvent resistance and, moreover, can be readily molded and designed compared to metals and woods, etc., those are rapidly entered into markets, and which have been widely employed in various fields such as wrapping materials, construction materials, and materials for cars and consumed in a large amount.

As molding methods for plastics, there can be applied various means such as vacuum molding, blow molding, and inflation molding, etc. depending upon uses thereof.

When plastics materials are considered from a viewpoint of the uses thereof, in the case of the uses for a film, polyethylenes and polypropylenes, etc. are mainly preferred, and in the case of sheets and vessels, polystyrenes and polypropylenes, etc. are usually preferred, and those are selectively employed in a variety of shapes depending upon uses. These are mainly selected in consideration of physical properties, price, and moldability in resins, etc.

As described hereinabove, kinds of plastics are usually selected depending upon uses, and a selection standard depends upon publicly-known various properties of the plastics. However, the publicly-known various properties are not fixed, and those can be occasionally modified by modification of molecular structure of the plastics, particularly, sterically structural modification, modification by chemical reaction, and modifications by addition of other resins or additives, which largely contribute to an enlargement in uses of the plastics.

There is large an effect by the use of plasticizer which is an additive, and it enlarges the uses of a variety of plastics. However, it is occasionally problematic that a limited plasticizer alone can be employed for a certain specified plastics, and that a preferred plasticizer is not still found and, a modification technology of the plastics by the use of the plasticizer is not still satisfied.

For example, if tensile elasticity of a polystyrene can be modified from 2x10₄-2.5x10⁴ kgf/cm² to a low value of less than 6x10³ kgf/cm², in addition to hard articles such as vessels and bottles in an existing circumstance, it is thought that there can be expected an enlargement in a use as a thin layer polystyrene film, and there can be also molded a mulch film for agriculture and a bag for kitchen garbages, etc.

In the case, as a means for adjusting the tensile elasticity, although there is a method for adjusting a molecular weight and a branched chain thereof, a delicate adjustment by the method is difficult and troublesome, and since an adjustable range is also narrow, it is not always a preferred means as the method for adjusting the above-described various properties.

### SUMMARY OF THE INVENTION

The present inventors of a first aspect of the present invention, as a result of an intensive investigation of a citrate compound concerning a plasticizer for thermoplastic resins, found out that a specified citrate compound is novel and can be preferably employed as a plasticizer for thermoplastic resins, and the present invention has been completed.

That is, according to the first aspect of the present invention, there is provided a citrate compound represented by general formula [1], 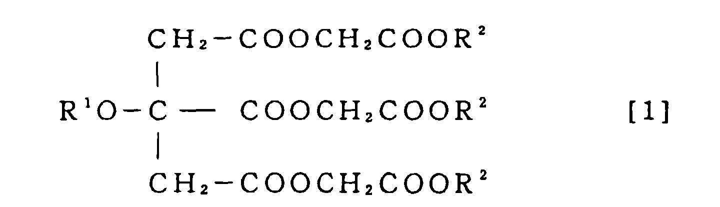 (in the formula, R¹ is a hydrogen atom or an aliphatic acyl group, and R² is an alkyl group).

Further, there is provided a citrate compound, wherein the R¹ is a hydrogen atom or an aliphatic acyl group having a carbon number of 1-5. Still further, there is provided a citrate compound, wherein the R² is an alkyl group having a carbon number of 1-4. Also, there is provided a citrate compound, wherein the R¹ is a hydrogen atom, and the R² is a methyl group or an ethyl group. Also, there is provided a citrate compound, wherein the R¹ is an acetyl group, and the R² is a methyl group or an ethyl group. Also, there is provided a plasticizer for thermoplastic resins comprising any one of the above-described citrate compounds. And also, there is provided a plasticizer for a cellulose acetate-based resin, a plasticizer for a vinylchloride-based resin, or a plasticizer for a styrene-based resin.

The present inventors of a second aspect of the present invention found that a citrate compound such as trisethoxy carbonylmethyl citrate and trisethoxy carbonylmethyl acetyl citrate can be employed as a plasticizer which does not cause a bleeding for the thermoplastic resins such as a cellulose acetate-based resin, a vinylchloride-based resin, or a styrene-based resin, and the present invention has been completed.

That is, according to the first aspect of the present invention, there is provided a thermoplastic resin composition which comprises a thermoplastic resin and the citrate compound represented by the general formula [1] of the first aspect of the invention. Also, there is provided a thermoplastic resin composition which comprises 100 parts by weight of a thermoplastic resin and 5-200 parts by weight of a citrate compound. Also, there is provided a thermoplastic resin composition wherein the thermoplastic resin is a cellulose acetate-based resin. Also, there is provided a thermoplastic resin composition wherein in the thermoplastic resin using a cellulose acetate-based resin, acetylation degree of the cellulose acetate-based resin is 40.03-62.55%.

Also, there is provided a thermoplastic resin composition wherein the thermoplastic resin is a vinylchloride-based resin. Also, there is provided a thermoplastic resin composition wherein the thermoplastic resin is a styrene-based resin. Further, there is provided a card prepared from a cellulose acetate-based resin which comprises a cellulose acetate-based resin and a plasticizer which does not cause bleeding from the cellulose acetate-based resin. Still further, there is provided a card prepared from a cellulose acetate-based resin wherein the plasticizer is a citrate compound represented by general formula [2] described below. 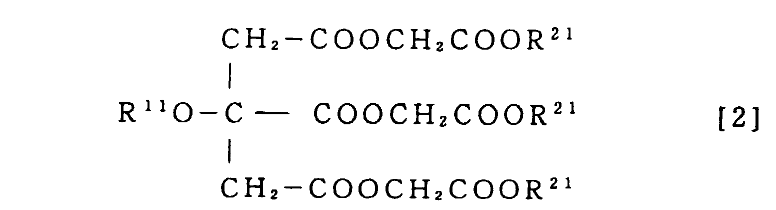 (in the formula, R¹¹ is a hydrogen atom or an aliphatic acyl group having a carbon number of 1-5, and R²¹ is an alkyl group having a carbon number of 1-4).

Also, there is provided a card prepared from a cellulose acetate-based resin wherein the plasticizer is trisethoxy carbonylmethyl citrate, acetyl trisethoxy carbonylmethyl acetyl citrate, and a mixture thereof. Also, there is provided a card prepared from a cellulose acetate-based resin which comprises 100 parts by weight of a cellulose acetate-based resin and 5-200 parts by weight of the plasticizer. And also, there is provided a card prepared from a cellulose acetate-based resin which comprises 100 parts by weight of a cellulose acetate-based resin and less than 40 parts by weight of the plasticizer, wherein weight retention ratio of the card is more than 99% after thermally drying (at 80°C in a hot air-circulating dryer for 24 hours).

### BRIEF DESCRIPTION OF DRAWINGS

Figure 1 is an NMR chart of a citrate compound in Example 1-1.

Figure 2 is an IR chart of a citrate compound in Example 1-1.

Figure 3 is an NMR chart of a citrate compound in Example 1-3.

Figure 4 is an IR chart of a citrate compound in Example 1-3.

Figure 5 is an NMR chart of a citrate compound in Example 1-4.

Figure 6 is an IR chart of a citrate compound in Example 1-4.

### DETAILED DESCRIPTION OF THE INVENTION

Hereinafter, a first aspect of the invention is illustrated in detail.

### <Citrate compound>

In the novel citrate compound represented by the general formula [I] which is provided in the first aspect of the invention, R¹ is a hydrogen atom or an aliphatic acyl group and, although R¹ which is an aliphatic acyl group is not particularly limited, a carbon number is preferably 1-12, and more preferably 1-5. Specifically, there can be exemplified formyl, acetyl, propionyl, butyryl, valeryl, parmitoyl, and oleil, etc., R² which is an alkyl group is not particularly limited, which may have even a linear chain or branched chain, and it is preferably an alkyl group having a carbon number of 1-24, and more preferably 1-4. Specifically, there can be exemplified methyl group, ethyl group, n-propyl group, isopropyl group, n-butyl group, isobutyl group, sec-butyl group, and t-butyl group, etc.

Particularly, in a preferred plasticizer for the cellulose acetate-based resin, R¹ is a hydrogen atom, and R² is a methyl group or an ethyl group, and R¹ is an acetyl group, and R² is a methyl group or an ethyl group.

### <Method for the preparation of a citrate compound having a hydrogen atom as R¹>

Of the citrate compound of the present invention, one having a hydrogen atom as the R¹ can be prepared by applying a publicly-known method. As the publicly-known method, there can be enumerated a method in which a phthalyl glycolate is prepared from a phthalic half ester and an α-halogenated alkyl acetate which is described in, for example, British Patent No. 931,781.

Specifically, there are employed tri-sodium citrate, tri-potassium citrate, and citric acid (hereinafter, these are named a citric acid raw material) and, preferably, 1 mol of tri-sodium citrate is allowed to react with a larger amount than the chemical equivalent amount, preferably 1-10 mol, and more preferably 2-5 mol of α-monohalogenated alkyl acetate which is an alkyl ester corresponding to the R², for example, monochloromethyl acetate or monochloroethyl acetate.

In the case that moisture exists in a chemical reaction system, since yield of a desired compound lowers, there are employed raw materials containing as low moisture as possible. In the reaction, there can be employed catalysts such as trimethylamine, triethylamine, tri-n-propylamine, tri-isopropylamine, tri-n-butylamine, tri-iso-butylamine, and dimethyl cyclohexylamine, etc. which are a linear chain or cycloaliphatic tertiary amine. Of those, triethylamine is preferred. Use amount of the catalysts ranges in 0.01-1.0 mol, and preferably 0.2-0.5 mol based on 1 ml of raw materials for the citrate compound. Reaction is conducted at temperature of 60-150°C for 1-24 hours. Although solvents for the reaction are not always required, there can be employed toluene, benzene, xylene, and methylethyl ketone, etc. After completion of the reaction, by-products and catalysts are removed by adding, for example, water, and an oily layer is washed by water and then a desired product can be isolated from unreacted raw materials for the citrate compound by distillation.

### <Method for the preparation of a citrate compound having an aliphatic acyl group as the R¹>

The citrate compound of the present invention in which the R¹ is an aliphatic acyl group and the R² is an alkyl group can be prepared using the above-described citrate compound in which R¹ is hydrogen atom. That is, 1 mol of the citrate compound is allowed to react with 1-10 mol of a halogenated acyl, for example, halogenated formyl or acetyl chloride, etc. from which there is introduced the R¹ which is the aliphatic acyl group. As catalysts, a basic compound such as pyridine can be employed in an amount of 0.1-2 mol based on 1 mol of the citrate compound. Reaction may be conducted at the absence of solvents and at 80-100°C for 1-5 hours. After the completion of the reaction, water and a water-insoluble organic solvent such as, for example, toluene are added into a reaction mixture to dissolve a desired product into the organic solvent, followed by separating an organic solvent layer from a water layer and washing the organic solvent layer using water, and then isolating the desired product by a common method such as distillation.

### <Plasticizer for a thermoplastic resin which comprises a novel citrate compound>

The novel citrate compound provided in the present invention is added into various thermoplastic resins, and it functions as a plasticizer which is excellent in compatibility, non-volatility, and a non-migration property, etc.

The thermoplastic resins for which the novel citrate compound of the present invention can be employed as a plasticizer are not particularly limited, and general-purpose plastics can be widely employed. For example, there are included cellulose acetate-based resins, vinylchloride-based resins, styrene-based resins, olefine-based resins such as polyethylenes, ethylene-vinyl acetate copolymers, polypropylenes, and ethylene-propylene copolymers, ester-based resins such as polyethylene terephthalates and polybutylene terephthalates, and polyamide-based resins such as nylon 46, nylon 6, nylon 66, nylon 11, and nylon 12, etc. Of those, there are preferably employed the cellulose acetate-based resins, vinylchloride-based resins, and styrene-based resins.

The cellulose acetate-based resins include all ones in which at least one of hydroxyl groups in a cellulose chain are acetylated. Specifically, there are enumerated a cellulose acetate, a cellulose acetate propionate, a cellulose acetate butylate, and a cellulose acetate phthalate, etc., and the cellulose acetate, cellulose acetate propionate, and cellulose acetate butylate are preferred, and the cellulose acetate is more preferred.

Of the cellulose acetate-based resins, there are more preferred ones having acetylation degree of 40.03-62.55%, that is, substituted degree of 1.5-3.0 per repeated units in the cellulose, and there are particularly preferred ones having acetylation degree of 50-61%.

Amount of the citrate compound to be added to the cellulose acetate-based resins is preferably 5-200 parts by weight based on 100 parts by weight of the cellulose acetate-based resins and, particularly, 10-200 parts by weight. From a viewpoint of mechanical properties and processability, etc., it is more preferred in 20-100 parts by weight. In the amount of the citrate compound exceeding 5 parts by weight, a function as a plasticizer becomes remarkable and, in the amount of the citrate not more than 200 parts by weight, a function as a plasticizer becomes maximum, and it is an economical amount to be added.

The vinylchloride-based resins are a polymeric resin containing vinylchloride monomer units in polymers which are usually employed, and which include, for example, a vinylchloride homopolymer and a polyvinylchloride containing a slight amount of vinylacetate and vinylidene chloride, etc. as a copolymerizable component. The citrate compound to be employed in the present invention is shown by the general formula [1] which is illustrated hereinabove, and there is particularly preferred one having hydrogen or acetyl group as the R¹ and methyl group or ethyl group as the R² for formulating in the vinylchloride-based resins.

Amount of the citrate compound to be added to the vinylchloride-based resins is preferably 5-200 parts by weight based on 100 parts by weight of the vinylchloride-based resins. It is more preferably 7-30 parts by weight in view of mechanical properties and moldability, etc. In the case that the mount of the citrate compound is less than 5 parts by weight, a function as a plasticizer is not shown at all and, contrarily, in the case of exceeding 200 parts by weight, a function as a plasticizer does not increase at all, resulting in becoming an unnecessary addition amount.

As the styrene-based resins, there can be enumerated a styrene-based resin such as a general-purpose polystyrene, an impact-resistible polystyrene, an AS resin, and an ABS resin, etc. The citrate compound to be employed in the present invention is shown by the general formula [1] which is illustrated hereinabove, and there are particularly preferred one which has an aliphatic acyl group having a carbon number of 1-5 as the R¹, and an alkyl group having a carbon number of 1-4 as the R², one which has acyl group having a hydrogen atom as the R¹, and a methyl group or ethyl group as the R², and one which has an acetyl group having as the R¹, and a methyl group or ethyl group as the R² for formulating in the styrene-based resins.

Amount of the citrate compound to be added to the styrene-based resins is preferably 5-200 parts by weight based on 100 parts by weight of the styrene-based resins to obtain a thermoplastic resin. The amount of the citrate compound is more preferably 20-100 parts by weight in view of processability such as moldability and mechanical properties of a molded article, etc. In the case that the mount of the citrate compound is less than 5 parts by weight, a function as a plasticizer is not shown at all and, contrarily, in the case of exceeding 200 parts by weight, a function as a plasticizer does not increase at all, resulting in becoming an unnecessary addition amount.

The citrate compound is added to the thermoplastic resins as a plasticizer, and those are employed after mixing.

A method for kneading is not particularly limited, and a means which is usually employed in kneading for thermoplastic resins can be applied without any limitations. For example, those are kneaded at a melting temperature using an extruder (a single screw or twin screw), a kneader, a Banbury mixer, and a roll, etc., or kneaded by dissolving those using an appropriate solvent. Since the citrate compound of the present invention is not apt to cause bleeding from the thermoplastic resins, it can be employed as a plasticizer which is improved in kneading workability.

Hereinafter, a second aspect of the invention is illustrated in detail.

The second aspect of the present invention provides a thermoplastic resin composition which comprises a thermoplastic resin and the citrate compound of the above-described first aspect of the invention. The citrate compound to be employed in the present invention is the citrate compound shown by the general formula [1] illustrated in the above-described first aspect of the invention. Method for the preparation thereof is also the same as illustrated in the above-described first aspect of the invention. As the thermoplastic resins, there can be employed general-purpose thermoplastic resins illustrated in the above-described first aspect of the invention and, of those, there can be preferably employed the cellulose acetate-based resins, vinylchloride-based resins, and styrene-based resins.

The thermoplastic resins are illustrated in the above-described first aspect of the invention. Further, a formulating proportion of the thermoplastic resins with the citrate compound can be also applied which is illustrated in the above-described first aspect of the invention.

In order to obtain the thermoplastic resin composition of the present invention in which the citrate compound is added to the thermoplastic resins, a method for mixing both is not particularly limited and, a means which is usually employed in kneading for thermoplastic resins can be applied without any limitations. For example, those are kneaded at a melting temperature using an extruder (a single screw or twin screw), a kneader, a Banbury mixer, and a roll, etc., or kneaded by dissolving those using an appropriate solvent.

In the thermoplastic resin composition of the present invention, as well as in the case of usual resin compositions for molding which have been conventionally known, there may be optionally added other additives, for example, a variety of stabilizers such as anti-oxidants and ultraviolet ray absorbents, dyes, pigments, fillers, lubricants, anti-static agents, and flame retardants, etc. which may be added solely or in combination.

As one of the second aspects of the invention, there is a card made from the cellulose acetate-based resin composition containing a plasticizer which does not cause bleeding from the cellulose acetate-based resin. The card has biodegradability and same physical properties as general-purpose resins.

There is employed a citrate compound which does not cause bleeding with a lapse of time even though it is melted and kneaded after adding to the cellulose acetate-based resin as a plasticizer.

The citrate compound which does not cause bleeding from the cellulose acetate-based resin means a citrate compound having a weight retention ratio in the card made from the cellulose acetate-based resin of not less than 99.5%, not less than 99%, and not less than 95%, and preferably, not less than 99.9%, not less than 99.5%, and not less than 97%, respectively, in the case that it is formulated in 20 parts by weight, 40 parts by weight, and 100 parts by weight based on 100 parts by weight of the cellulose acetate-based resin. The weight retention ratio is measured after drying while heating (80°C, for 24 hours in a hot air circulating dryer).

Specifically, there can be employed, for example, the citrate compound represented by the above-described general formula [2]. Of those, there can be preferably employed trisethoxycarbonylmethyl citrate in which R¹ is a hydrogen atom and R² is an ethyl group, trisethoxycarbonylmethyl acetyl citrate in which R¹ is an acetyl group and R² is an ethyl group, and a mixture thereof.

The cellulose acetate-based resin to be employed in the present invention is not particularly limited, and there are enumerated a cellulose acetate, a cellulose acetate-propionate, a cellulose acetate-butyrate, and a cellulose acetate-phthalate, etc., and there are usefully employed a cellulose acetate, a cellulose acetate-propionate, and a cellulose acetate-butyrate from a viewpoint of physical properties of a card after molding and biodegradability. Of the above-described cellulose acetate-based resins, there is preferred a cellulose acetate-based resin having an acetylated degree of 40.03-62.55%, that is, there is more preferred a cellulose acetate-based resin having a substituted degree of 1.5-3.0 per a repeated unit of cellulose, and there is particularly preferred a cellulose acetate-based resin having an acetylated degree of 50-61%.

A preferred mode of the present invention is a card made from a cellulose acetate-based resin having the weight retention ratio of not less than 99% when a card made from a cellulose acetate-based resin containing not more than 40 parts by weight of the plasticizer based on 100 parts by weight of the cellulose acetate-based resin is thermally dried at 80°C for 24 hours in a hot-air circulating dryer.

Formulation proportion of the plasticizer is 5-200 parts by weight, preferably 10-200 parts by weight, more preferably 20-100 parts by weight, and most preferably 20-60 parts by weight based on 100 parts by weight of the cellulose acetate-based resin.

In the resin composition to be employed for preparing the card containing the cellulose acetate-based resin and the plasticizer, a variety of additives can be optionally added.

As the additives, there are enumerated anti-discoloring agents, anti-oxidants, lubricants, organic or inorganic pigments, and fillers, etc.

As the anti-discoloring agents, there are enumerated ADEKA STAB AO-70 which is a phenol-based agent and ADEKA STAB 2112 which is a phosphite-based agent which are manufactured by Asahi Denka, Ltd. Addition amount is 0.02-3 parts by weight and preferably 0.1-1.0 parts by weight based on 100 parts by weight of the cellulose acetate-based resin.

As the anti-oxidants, there are enumerated an amine-based one, a phenol-based one, a phosphorus-based one, and a sulphur-based one, etc. Addition amount is 0.02-3 parts by weight and preferably 0.1-1.0 parts by weight based on 100 parts by weight of the cellulose acetate-based resin.

As the lubricants, there are enumerated an aliphatic fatty acid amide-based one, an aliphatic fatty acid ester-based one, and a higher alcohol-based one, etc. Addition amount is 0.02-3 parts by weight and preferably 0.1-1.0 parts by weight based on 100 parts by weight of the cellulose acetate-based resin.

The fillers are preferably inorganic fillers, and there can be exemplified calcium carbonate, mica, calcium silicate, white carbon, finely-powdered silica, asbestos, clay, glass fiber, and a mixture thereof, etc. In the case that the fillers are fiber-state, flexural strength in extension direction is elevated. Addition amount is 0.5-50 parts by weight and preferably 5-10 parts by weight based on 100 parts by weight of the cellulose acetate-based resin.

In the case of kneading the cellulose acetate-based resin which is a base material for the card, the plasticizers, and the above-described various additives to be optionally added, there can be applied a method kneading at melting temperature and a method by dissolving and kneading in which an appropriate solvent is employed together. In kneading the above-described composition, there can be employed a kneading apparatus which is employed for kneading a usual thermoplastic resin and additives for resins such as a plasticizer without any limitation. As a kneader, there can be enumerated a single- or twin-screw extruder, a kneader, a Banbury mixer, and a roll, etc.

After kneading, the resin composition is optionally molded into a pellet shape, and pellets can be employed as a sheet for molding a card. Also for sheet preparation of the pellets, there can be employed an apparatus employed for sheet-preparation and film-preparation of usual thermoplastic resins and the above-described kneading apparatuses without any limitation.

Thus-obtained card made from the cellulose acetate-based resin can be widely utilized as a credit card and cashing card, etc. which are thick in the thickness, and a prepaid card and a telephone card, etc. which are thin in the thickness.

### [Examples and Comparative Examples]

Hereinafter, although the present inventions are more specifically illustrated by Examples and Comparative Examples, the present inventions are not limited to those.

It is to be noted that test methods for properties of the resin compositions in the first and second aspects of the invention are as follows.

### <Kneadability in melting>

The citrate compound of the present invention was added to a cellulose acetate resin in a variety of formulating amount using a small size melt kneader (Laboplastomill) to prepare a mixture. Approximately 50 g of the mixture was supplied to an evaluation test for the kneadability in melting.

Evaluation items are as follows from a viewpoint of workability through preparing in the case of a scale-up.

Those are an extent of torque (qualitatively shown "small", "middle", and "large"), occurrence of an order (qualitatively shown "exceedingly small", "middle", and "large"), occurrence of smoke (qualitatively shown "exceedingly small", "middle", and "large"), a liquid leakage state (shown by the presence or the absence), an interlocking property of a resin into a screw (qualitatively shown ⓞ, O, Δ, and ×), and a discoloration state (shown by color observed), and a plasticizing effect (qualitatively shown O, Δ, and ×).

It is to be noted that "liquid leakage" in the above-described evaluation items means a phenomenon that an admixture and decomposed products therefrom ooze out from connecting portions of the Laboplastomill which is an assembled apparatus.

Kneading conditions are as follows.
(1) Kneading temperature: 220°C
(2) Kneading time of period: 10 minutes
(3) Screw revolution: 50 rpm

### <Bleeding property>

In relation to a molten admixture in which a plasticizing effect is shown in an evaluation test for a melt kneading property by a small size melt-kneading apparatus, there was conducted a bleeding property test (a test for a moving property with lapse of a time) of a plasticizer.

The test is a test for observing the presence or the absence of bleeding, and it was conducted by an evaluation of weight retention ratio, dimensional stability (volume retention ratio), and observation (visually) of an outer appearance.

A sheet having thickness of 0.5 mm was molded by feeding the above-described molten admixture into a hot press machine under conditions of a compression temperature of 220°C, compression pressure of 220 kgf/cm², compression time and cooling time of period of 3 minutes, respectively. The sheet was cut into a square size of 5 cm x 5 cm to prepare test pieces. The test pieces were employed in Examples 1-5 to 1-7 and Comparative Examples 1-1 and 1-2.

Further, a color plate (90 mm x 50 mm x a three-stage thickness of 1 mm, 2 mm, and 3 mm, respectively) was molded from a molten admixture prepared by a twin screw extruder for Example 1-8 and Comparative Example 1-3.

In other Examples and Comparative Examples, color plates identically obtained by an injection were used for the tests.

Experimental conditions in the tests of the bleeding property are as follows. After the tests of the bleeding property, there were evaluated weight retention ratio, dimensional stability (volume retention ratio), and observation (visually) of an outer appearance.
(1) Test by a hot air circulation dryer: 80°C (24 hours)
(2) Test by a dryer maintained at a constant temperature and humidity: 50°C x 80% RH (24 hours)

The weight retention ratio is represented by percentage of weight ratio of the test pieces before and after the tests in which there was measured the weight in the test pieces having thickness of 0.5 mm cut into square size of 5 cm x 5 cm for the tests of the bleeding property and the color plates molded by injection, and then, those were treated by the above-described conditions, and the surface of the test pieces was wiped by gauze, followed by measuring the weight.

### <Tensile test>

As test pieces to be employed in Examples 1-5 to 1-7 and Comparative Examples 1-1 to 1-2, there was employed a test piece according to JIS K7113 except that there was employed a rectangular piece having the width of 10 mm by cutting a compressed plate having the thickness of 0.5 mm prepared by the same molding conditions as in the sheet molded for the above-described tests of the bleeding property.

As test pieces to be employed in Example 1-8 to Comparative Example 1-3, there was employed JIS No. 1 tensile test piece according to JIS K7113 obtained through an injection molding by employing the same admixture as well as in the above-described tests of the bleeding property. It is to be noted that in the above all cases, test pieces were supplied to the tests after controlling humidity at 23°C x 50% RH for 24 hours in a dryer maintained at a constant temperature and humidity.

### <Flexural test, Izod impact test, heat distortion temperature test, and color hue test>

(1) Flexural test: It was conducted according to JIS K7203.
(2) Izod impact test: It was conducted according to JIS K7110.
(3) Heat distortion temperature test: It was conducted according to JIS K7207.
(4) Color hue test: It was conducted according to JIS K7103. YI is a yellowing index.

### Examples in the first aspect of the Invention

### <Example 1-1/Preparation Example of a compound having R¹ of a hydrogen and R² of a methyl group>

A four-necked flask was equipped with an agitator, a thermometer, and a condenser, and charged with 774.4 g (3.0 mol) of trisodium citrate, 26.5 g (0.26 mol) of triethyl amine, and 1172 g (10.8 mol) of monochloromethyl acetate, followed by heating to 90°C while agitating and allowing to react at 90°C for 15 hours. After the completion of the reaction, the flask was cooled to 80°C, and 1500 g of water was added to remove sodium chloride by-produced. An oily layer was thrice washed with 500 g of water, and monochloromethyl acetate was recollected by vacuum distillation at 90°C and 40 mmHg, followed by conducting water vapor distillation at 90°C and 40 mmHg for 1 hour to obtain 978 g of a final product.

In the final product, the specific gravity is 1.360 and a purity by a gel permeation chromatography (GPC: HLC-8020 manufactured by Toso, Ltd.) is 74.5%.

The final product was assigned by results measured using ¹³C-NMR. From chart, there was identified the presence of a peak by a quaternary carbon (citric acid structure) at 73.306 ppm, a peak by methylene group connected to the quaternary carbon at 42.591 ppm, a peak by carbonyl group connected thereto at 169.002 ppm, a peak by methylene group sandwiched between carbonyl groups connected thereto at 60.984 ppm, a peak by carbonyl group connected thereto at 67.545 ppm, and a peak by methyl group connected thereto at 52.394 ppm.

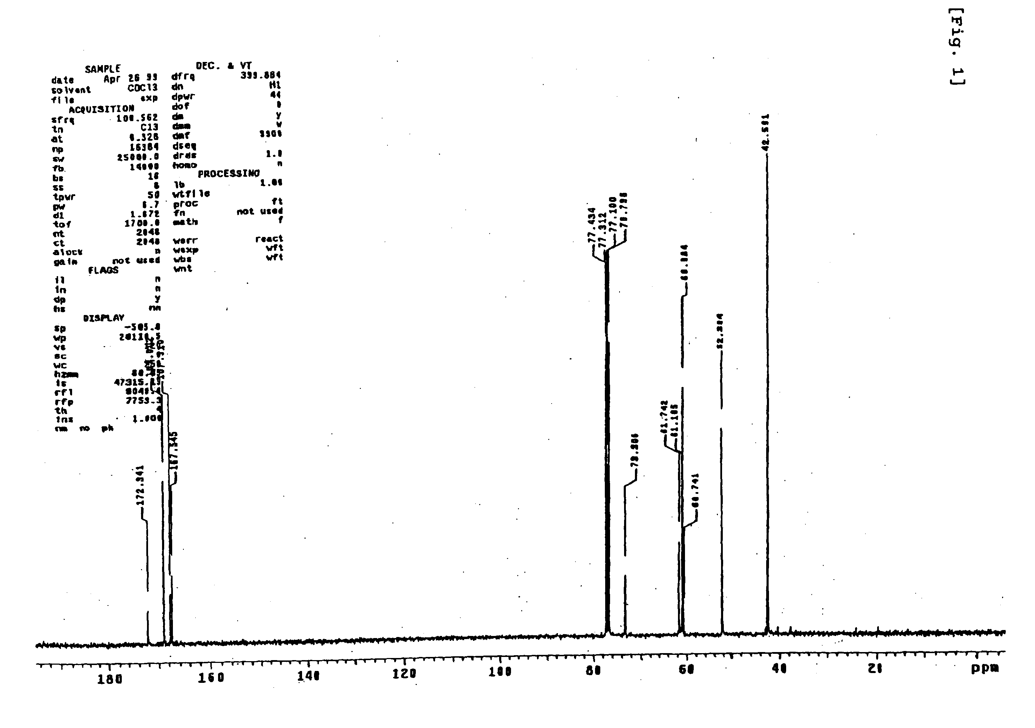

Further, there was also identified the presence of a peak by carbonyl group connected to a quaternary carbon at 172.341 ppm, a peak by methylene group connected thereto at 61.742 ppm, a peak by carbonyl group connected thereto at 167.910 ppm, and a peak by methyl group connected thereto at 52.394 ppm. From the results, it was confirmed that a structure of the citrate is shown by structural formula (1) described below. Figure 1 shows the NMR chart.

Still further, from results of an FT-IR measurement, there is suggested the presence of a stretching vibration by C=O at 1747.4 cm⁻¹, a stretching vibration by O-H at 3500.8 cm⁻¹ and a stretching vibration by C-H at 2850-3000 cm⁻¹.

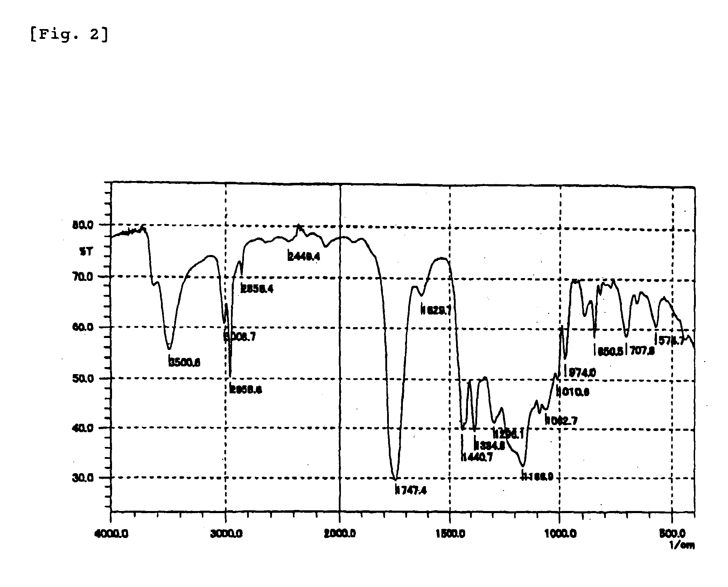

Figure 2 shows the IR chart.

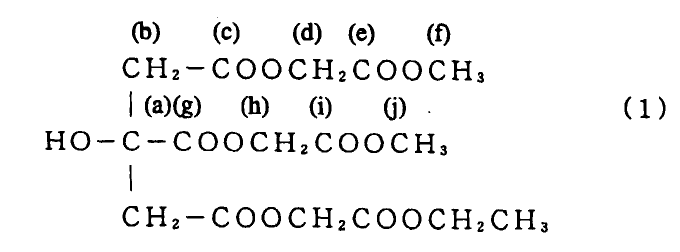

(a): 73.306 ppm, (b): 42.591 ppm, (c): 169.002 ppm

(d): 60.984 ppm, (e): 167.545 ppm, (f): 52.394 ppm

(g): 172.341 ppm, (h): 61.742 ppm, (i): 167.910 ppm

(j): 52.394 ppm

In the Figure 2 (IR chart), measuring conditions, etc. are as follows.
S-3-2. IRS: April 02, 1999
Calculated times: 20
Type: HYPER IR User: Shimadzu customer
Detector: standard
Horizontal coordinate: 1/cm Vertical coordinate: %T
Abodize function: Happ
Minimum Wave Number: 401.17 Maximum Wave Number: 3998.16
Wave Number scope: 1/cm
Data Points: 1868 Data Interval: 1.92868
Resolution: 4.0
Gain: auto Aperture: auto
Miller Speed: 2.8
S-3-2. Results of peak detection by IRS: 17 peaks
Threshold Value: 80 Noise Level: 3
Wave Number Scope: unfixed

| No. | Position (1/cm) | Intensity (%T) |
|---|---|---|
| 1 | 574.7 | 60.544 |
| 2 | 707.8 | 58.436 |
| 3 | 850.5 | 58.213 |
| 4 | 974.0 | 54.038 |
| 5 | 1010.6 | 50.107 |
| 6 | 1062.7 | 43.985 |
| 7 | 1166.9 | 32.379 |
| 8 | 1296.1 | 41.177 |
| 9 | 1384.8 | 39.472 |
| 10 | 1440.7 | 39.684 |
| 11 | 1629.7 | 66.342 |
| 12 | 1747.4 | 29.408 |
| 13 | 2449.4 | 77.123 |
| 14 | 2858.4 | 70.432 |
| 15 | 2958.6 | 50.218 |
| 16 | 3008.7 | 60.824 |
| 17 | 3500.6 | 55.613 |

### <Example 1-2/Preparation Example of a compound having R¹ of a hydrogen and R² of an ethyl group>

A four-necked flask was equipped with an agitator, a thermometer, and a condenser, and charged with 774.4 g (3.0 mol) of nonhydrated trisodium citrate, 26.5 g (0.26 mol) of triethyl amine, and 1323 g (10.8 mol) of monochloroethyl acetate, followed by heating to 120°C while agitating and allowing to react at 120°C for 7 hours. After the completion of the reaction, the flask was cooled to 80°C, and 1500 g of water was added to remove sodium chloride by-produced. An oily layer was thrice washed with 500 g of water, and monochloroethyl acetate was recollected by vacuum distillation at 100°C and 45 mmHg, followed by conducting water vapor distillation at 100°C and 40 mmHg for 1 hour to obtain 1284 g of a final product.

In the final product, the specific gravity is 1.280 and a purity by a gel permeation chromatography (GPC: HLC-8020 manufactured by Toso, Ltd.) is 88.0%.

### <Example 1-3/another Preparation Example of a compound having R¹ of a hydrogen and R² of an ethyl group>

A four-necked flask was equipped with an agitator, a thermometer, and a condenser, and charged with 774.4 g (3.0 mol) of nonhydrated trisodium citrate, 11 g (0.11 mol) of triethyl amine, and 1103 g (9.0 mol) of monochloroethyl acetate, followed by heating to 80°C while agitating and allowing to react at 80°C for 3 hours. After that, reaction temperature was gradually raised, followed by allowing to react at 120°C for 8 hours. After the completion of the reaction, the flask was cooled to 70°C, and 200 g of toluene and 1400 g of water were added to remove sodium chloride by-produced. An oily layer was thrice washed with 500 g of water to obtain 1450 g of a toluene solution of a final product.

Toluene was recollected by vacuum distillation at 100°C and 45 mmHg for 1 hour, followed by conducting water vapor distillation at 100°C and 40 mmHg for 1 hour to obtain 1050 g of a final product.

In the final product, the specific gravity is 1.265 and a purity by a gel permeation chromatography (GPC: HLC-8020 manufactured by Toso, Ltd.) is 93.1%.

The final product was assigned by results measured using ¹³C-NMR. From chart, there was identified the presence of a peak by a quaternary carbon (citric acid structure) at 73.306 ppm, a peak by methylene group connected to the quaternary carbon at 42.591 ppm, a peak by methylene group sandwiched between carbonyl groups connected thereto at 61.135 ppm, a peak by carbonyl group connected thereto at 167.090 ppm, a peak by methylene group connected thereto at 61.591 ppm, and a peak by methyl group connected thereto at 14.152 ppm.

Further, there were also identified a peak by carbonyl group connected to a quaternary carbon at 172.402 ppm, a peak by methylene group connected thereto at 61.894 ppm, a peak by carbonyl group connected thereto at 167.485 ppm, a peak by methylene group connected thereto at 61.651 ppm, and a peak by methyl group connected thereto at 14.152 ppm.

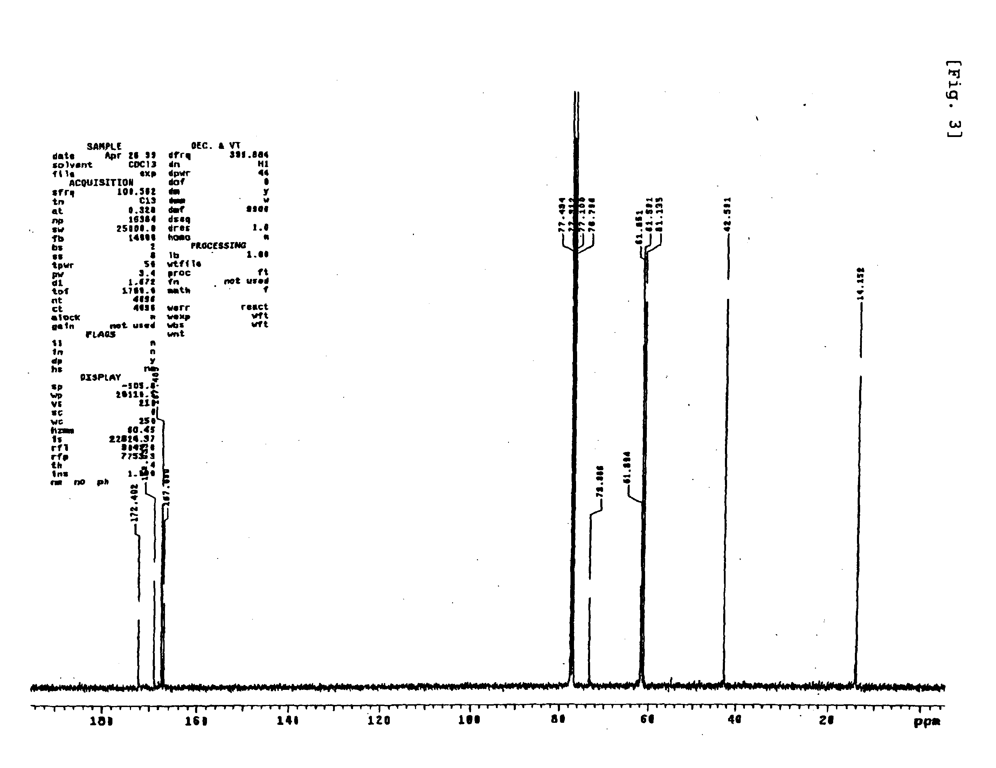

From the results, it was confirmed that a structure of the citrate is shown by structural formula (2) described below. Figure 3 shows the NMR chart.

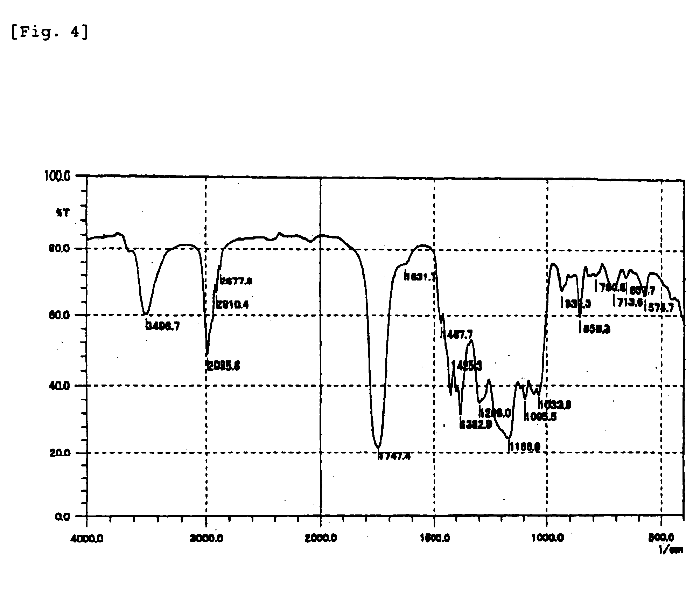

Still further, from results of an FT-IR measurement, there is suggested the presence of a stretching vibration by C=O at 1747.4 cm⁻¹, a stretching vibration by O-H at 3496.7 cm⁻¹ and a stretching vibration by C-H in the vicinity at 2870-2990 cm⁻¹. Figure 4 shows the IR chart.

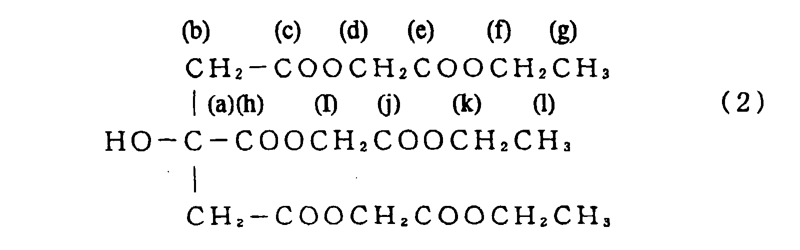

(a): 73.306 ppm, (b): 42.591 ppm, (c): 169.033 ppm

(d): 61.135 ppm, (e): 167.090 ppm, (f): 61.591 ppm

(g): 14.152 ppm, (h): 172.402 ppm, (i): 61.894 ppm

(j): 167.485 ppm (k): 61.651 ppm, (l): 14.152 ppm

In the Figure 4 (IR chart), measuring conditions, etc. are as follows.
S-2.IRS: April 02, 1999
Time: 17:03:57 Calculated times: 20
Type: HYPER IR User: Shimadzu customer
Detector: standard
Horizontal coordinate: 1/cm Vertical coordinate: %T
Abodize function: Happ
Minimum Wave Number: 401.17 Maximum Wave Number: 3998.16
Wave Number scope: 1/cm
Data Points: 1866 Data Interval: 1.92868
Resolution: 4.0
Gain: auto Aperture: auto
Miller Speed: 2.8
S-2.IRS: April 02, 1999
Date: June 12, 1999 Time: 17:03:57
Calculated times: 20
Type: HYPER IR User: Shimadzu customer
Detector: standard
Horizontal coordinate: 1/cm Vertical coordinate: %T
Abodize function: Happ
Minimum Wave Number: 401.17 Maximum Wave Number: 3998.16
Wave Number scope: 1/cm
Data Points: 1866 Data Interval: 1.92868
Resolution: 4.0
Gain: auto Aperture: auto
Miller Speed: 2.8
S-2. Results of peak detection by IRS: 19 peaks
Threshold Value: 80 Noise Level: 2
Wave Number Scope: unfixed

| No. | Position (1/cm) | Intensity (%T) |
|---|---|---|
| 1 | 574.7 | 66.154 |
| 2 | 657.7 | 71.396 |
| 3 | 713.6 | 67.918 |
| 4 | 790.8 | 72.154 |
| 5 | 858.3 | 59.559 |
| 6 | 939.3 | 67.335 |
| 7 | 1033.8 | 37.366 |
| 8 | 1095.5 | 35.991 |
| 9 | 1166.9 | 24.481 |
| 10 | 1298.0 | 35.086 |
| 11 | 1382.9 | 31.635 |
| 12 | 1425.3 | 38.117 |
| 13 | 1467.7 | 57.721 |
| 14 | 1631.7 | 75.591 |
| 15 | 1747.4 | 21.407 |
| 16 | 2877.6 | 73.930 |
| 17 | 2910.4 | 66.839 |
| 18 | 2985.6 | 48.484 |
| 19 | 3496.7 | 60.130 |

### <Example 1-4/Preparation Example of a compound having R¹ of an acetyl group and R² of an ethyl group>

A four-necked flask was equipped with an agitator, a thermometer, and a condenser, and charged with 150 g (0.333 mol) of the citrate prepared in the Example 1-2 and 27.7 g (0.345 mol) of pyridine, followed by cooling to 10°C while agitating. 27.1 g (0.345 mol) of acetyl chloride was further added from a dropping funnel over 30 minutes while cooling.

After that, reaction temperature was gradually raised, followed by allowing to react at 80°C for 2 hours. After the completion of the reaction, 200 g of toluene and 200 g of water were added to remove a hydrochloric salt of pyridine by-produced. An oily layer was thrice washed with 100 g of water to obtain 356 g of a toluene solution of a final product.

Toluene was recollected by vacuum distillation at 100°C and 45 mmHg, followed by conducting water vapor distillation at 110°C and 30 mmHg for 30 minutes to obtain 147 g of a final product. In the final product, the specific gravity is 1.264 and a purity by a gel permeation chromatography (GPC: HLC-8020 manufactured by Toso, Ltd.) is 87.8%.

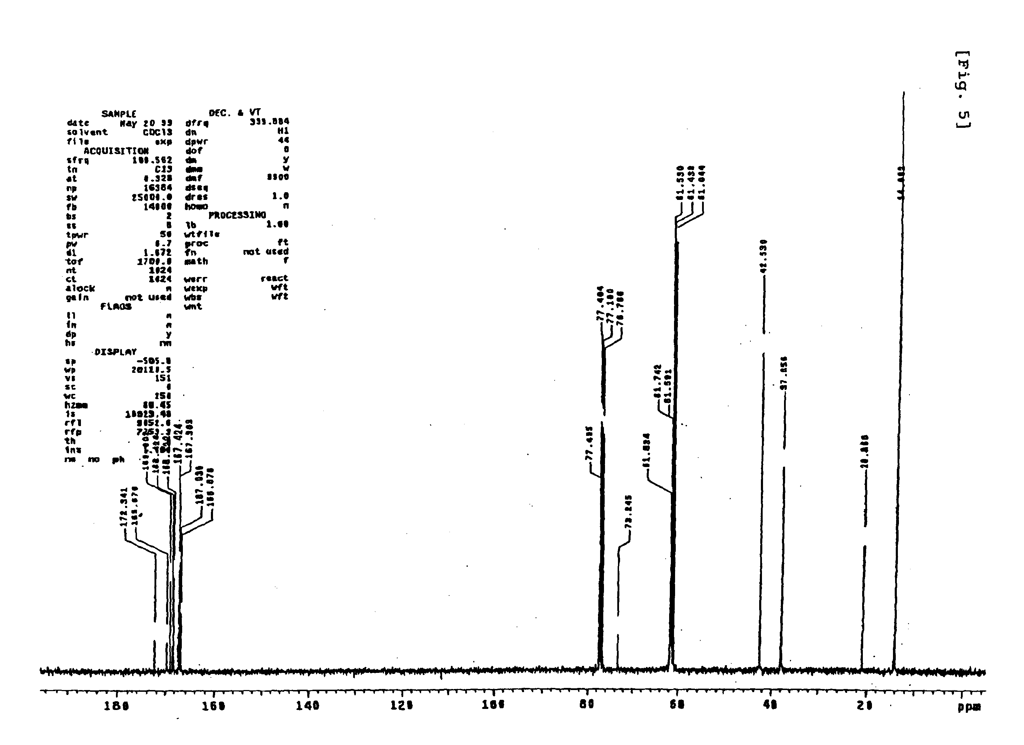

The final product was assigned by results measured using ¹³C-NMR. From the chart, there was identified the presence of a peak by a quaternary carbon (citric acid structure) at 73.245 ppm, a peak by methylene group connected to the quaternary carbon at 42.530 ppm, a peak by carbonyl group connected thereto at 169.002 ppm, a peak by methylene group connected thereto through oxygen at 61.044 ppm, a peak by carbonyl group connected thereto at 167.030 ppm, a peak by methylene group connected thereto at 61.439 ppm, and a peak by methyl group connected thereto at 14.092 ppm. Further, there was also identified the presence of a peak by carbonyl group connected to a quaternary carbon at 172.341 ppm, a peak by methylene group connected thereto through oxygen at 61.742 ppm, a peak by carbonyl group connected thereto at 167.424 ppm, a peak by methylene group connected thereto through oxygen at 61.530 ppm, and a peak by methyl group connected thereto at 14.092 ppm. Still further, there was also identified the presence of a peak by carbonyl group in acetyl group connected to a quaternary carbon at 169.670 ppm, and a peak by methyl group at 20.860 ppm. From the results, it was confirmed that a structure of the citrate is shown by structural formula (3) described below. Figure 5 shows the NMR chart.

Also, from results of an FT-IR measurement, there is suggested the presence of a stretching vibration by C=O at 1747.4 cm⁻¹ and a stretching vibration by C-H at 2870-2990 cm⁻¹.

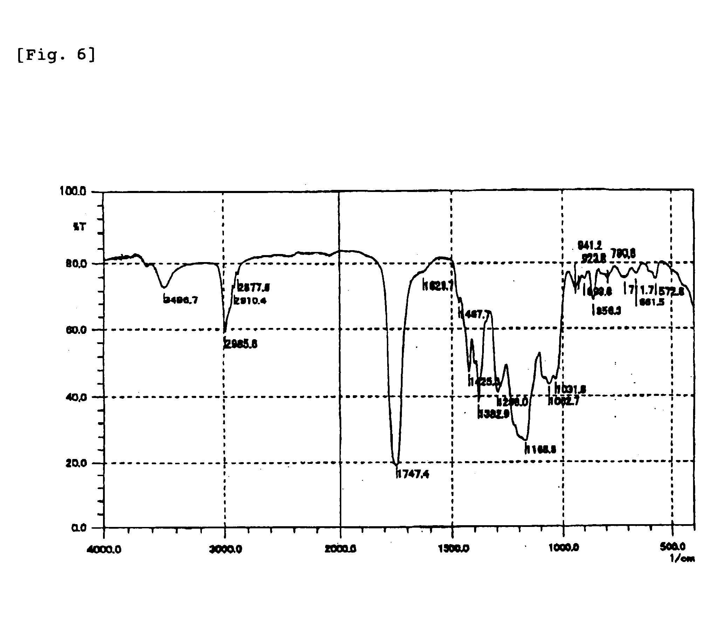

Figure 6 shows the IR chart. Because of a low purity, there is observed an absorption peak based on O-H stretching.

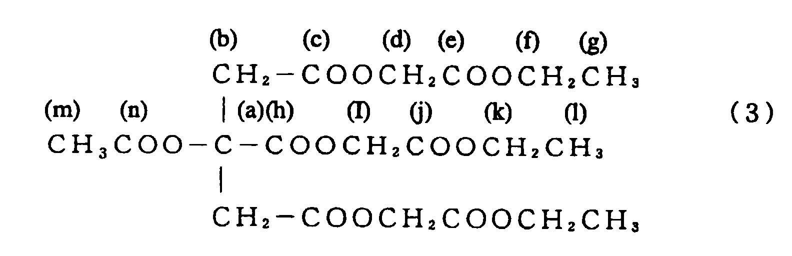

(a): 73.245 ppm, (b): 42.530 ppm, (c): 169.002 ppm

(d): 61.044 ppm, (e): 167.030 ppm, (f): 61.439 ppm

(g): 14.092 ppm, (h): 172.341 ppm, (i): 61.742 ppm

(j): 167.424 ppm (k): 61.530 ppm, (l): 14.092 ppm

(m): 20.860 ppm, (n): 169.670 ppm

In the Figure 6 (IR chart), measuring conditions, etc. are as follows.
S-2.IRS: April 02, 1999
Time: 13:23:54 Calculated times: 20
Type: HYPER IR User: Shimadzu customer
Detector: standard
Horizontal coordinate: 1/cm Vertical coordinate: %T
Abodize function: Happ
Minimum Wave Number: 401.17 Maximum Wave Number: 3998.16
Wave Number scope: 1/cm
Data Points: 1866 Data Interval: 1.92868
Resolution: 4.0
Gain: auto Aperture: auto
Miller Speed: 2.8
S-2. Results of peak detection by IRS: 21 peaks
Threshold Value: 80 Noise Level: 1.5
Wave Number Scope: unfixed

| No. | Position (1/cm) | Intensity (%T) |
|---|---|---|
| 1 | 572.8 | 75.410 |
| 2 | 661.5 | 70.713 |
| 3 | 711.7 | 75.835 |
| 4 | 790.8 | 75.594 |
| 5 | 856.3 | 69.051 |
| 6 | 898.8 | 75.397 |
| 7 | 923.8 | 74.167 |
| 8 | 941.2 | 72.717 |
| 9 | 1031.8 | 44.770 |
| 10 | 1062.7 | 43.534 |
| 11 | 1168.8 | 26.471 |
| 12 | 1298.0 | 41.082 |
| 13 | 1382.9 | 38.201 |
| 14 | 1425.3 | 47.439 |
| 15 | 1467.7 | 68.158 |
| 16 | 1629.7 | 77.312 |
| 17 | 1747.4 | 18.859 |
| 18 | 2877.6 | 76.983 |
| 19 | 2910.4 | 72.758 |
| 20 | 2985.6 | 59.077 |
| 21 | 3496.7 | 73.058 |

### <Example 1-5>

In proportion of 20 parts by weight, 40 parts by weight, and 100 parts by weight, respectively, trisethoxy carbonylmethyl citrate [the formula (2)] obtained in the above-described Example 1-3 was formulated with 100 parts by weight of a cellulose acetate resin having a weight average molecular weight of approximately 150,000 (hereinafter, merely referred to as molecular weight) and acetylated degree of 51%, followed by kneading with a small size melting kneading apparatus (Laboplastomill). Further, a compressed plate was prepared from a kneaded product with a hot press machine. Table 1-1 shows an evaluation result of kneading workability during kneading.

Tables 1-2 and 1-3 show experimental results of a tensile test and a bleeding property, respectively.

### <Example 1-6>

In proportion of 40 parts by weight, trismethoxy carbonylmethyl citrate [the formula (1)] obtained in the above-described Example 1-1 was formulated with 100 parts by weight of a cellulose acetate resin having a weight average molecular weight of approximately 150,000 and acetylated degree of 51%, followed by kneading with a small size melting kneading apparatus (Laboplastomill). Further, a pressed plate was prepared from a kneaded product with a hot press machine. Table 1-1 shows an evaluation result of kneading workability during kneading, and Tables 1-2 and 1-3 show experimental results of a tensile test and a bleeding property, respectively.

### <Example 1-7>

In proportion of 40 parts by weight, trismethoxy carbonylmethyl acetyl citrate [the formula (3)] obtained in the above-described Example 1-4 was formulated with 100 parts by weight of a cellulose acetate resin having a weight average molecular weight of approximately 150,000 and acetylated degree of 51%, followed by kneading with a small size melting kneading apparatus (Laboplastomill). Further, a pressed plate was prepared from a kneaded product with a hot press machine.

Table 1-1 shows an evaluation result of kneading workability during kneading, and Tables 1-2 and 1-3 show experimental results of a tensile test and a bleeding property, respectively.

### <Comparative Example 1-1>

In proportion of 40 parts by weight, a polycaprolactone oligomer having a molecular weight of 500 was formulated with 100 parts by weight of a cellulose acetate resin having a weight average molecular weight of approximately 150,000 and acetylated degree of 51%, followed by kneading with a small size melting kneading apparatus (Laboplastomill). Further, a pressed plate was prepared from a kneaded product with a hot press machine. Table 1-1 shows an evaluation result of kneading workability during kneading, and Tables 1-2 and 1-3 show experimental results of a tensile test and a bleeding property, respectively.

### <Comparative Example 1-2>

In proportion of 40 parts by weight, diethyl phthalate (DEP) was formulated with 100 parts by weight of a cellulose acetate resin having a molecular weight of approximately 150,000 and acetylated degree of 51%, followed by kneading with a small size melting kneading apparatus (Laboplastomill).

Further, a pressed plate was prepared from a kneaded product with a hot press machine. Table 1-1 shows an evaluation result of kneading workability during kneading, and Tables 1-2 and 1-3 show experimental results of a tensile test and a bleeding property, respectively.

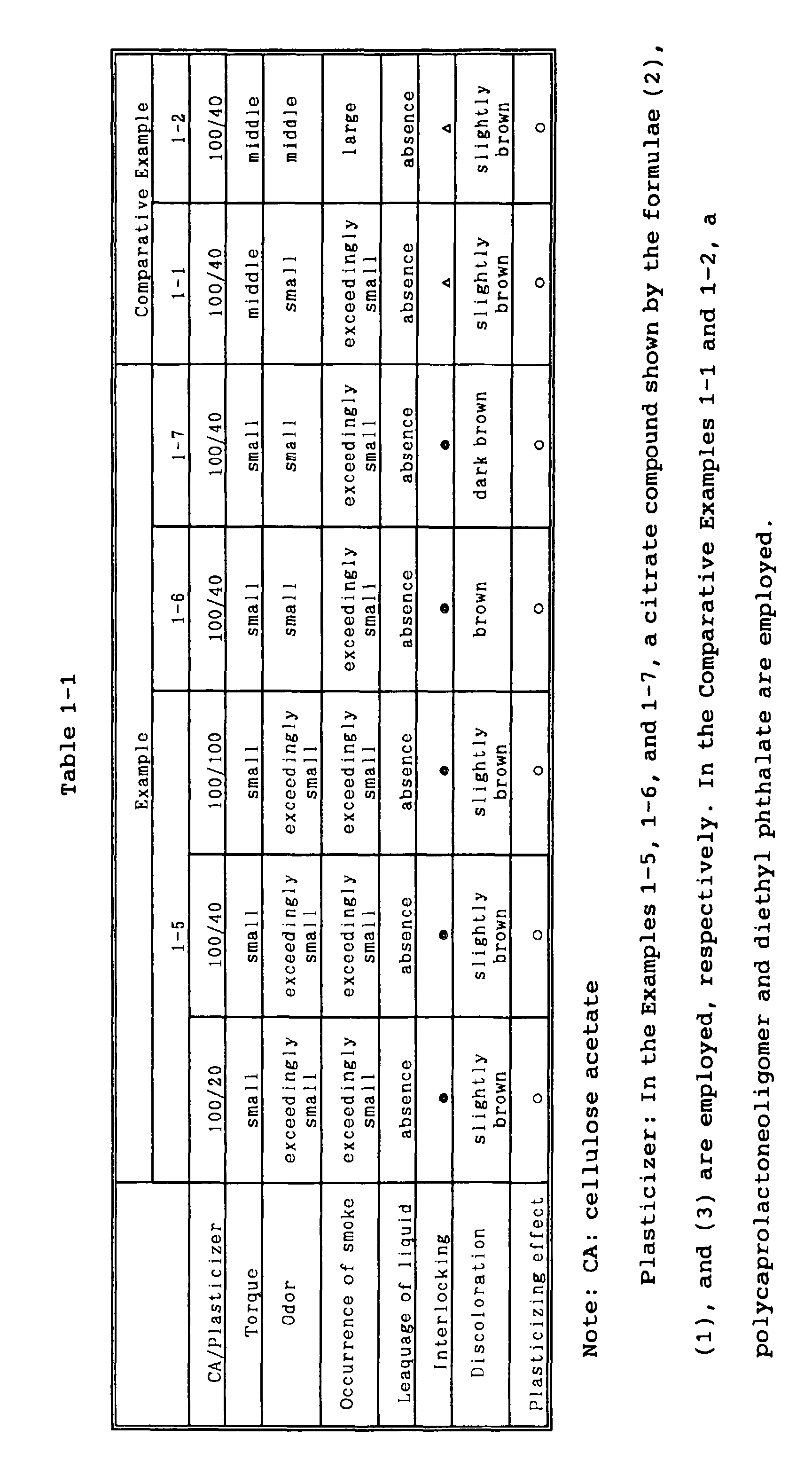

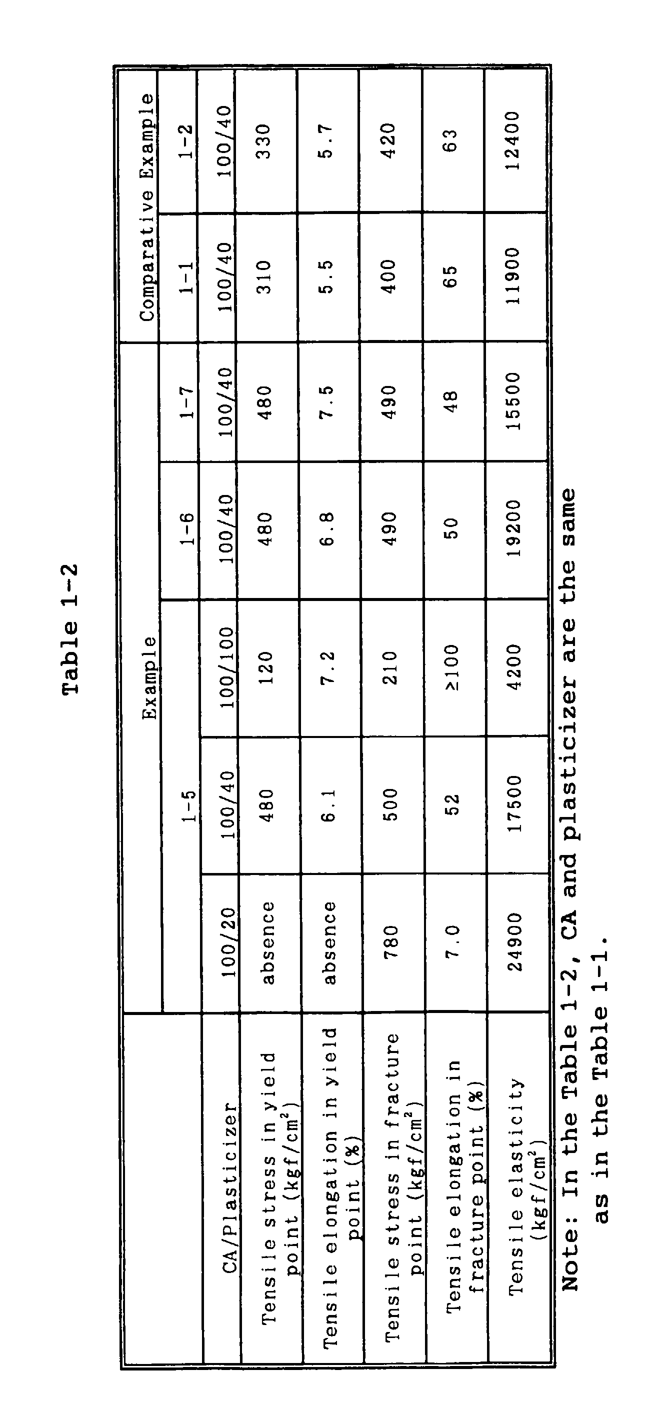

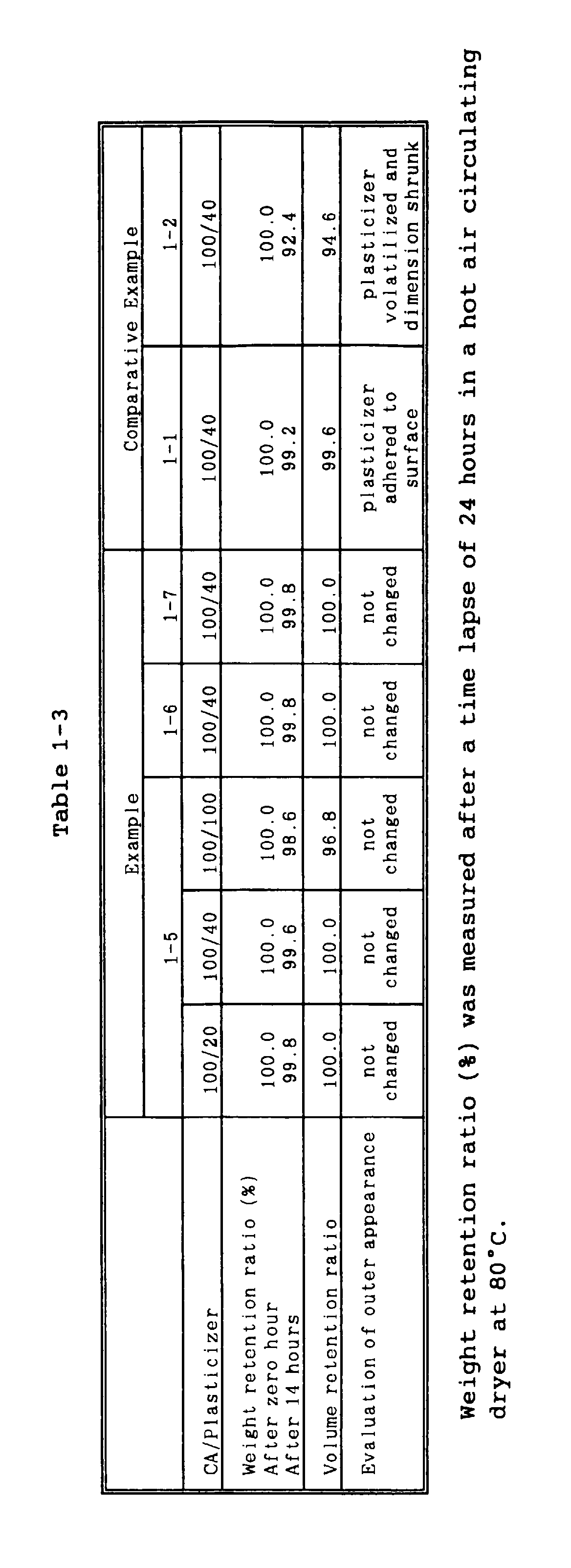

### <Example 1-8>

In proportion of 40 parts by weight, trisethoxy carbonylmethyl citrate employed in the Example 1-5 was formulated with 100 parts by weight of a cellulose acetate resin having a molecular weight of approximately 150,000 and acetylated degree of 51%, followed by kneading with a twin screw melting extruder to prepare pellets. Further, color plates for evaluation of physical properties were molded from the pellets using an injection machine. As evaluation tests, there were conducted a tensile test, a flexural test, an Izod impact strength test, a heat distortion temperature test, and a color hue test. Table 1-4 shows results. Further, a bleeding test was likewise conducted using the color plates. Table 1-5 shows results.

### <Comparative Example 1-3>

In proportion of 40 parts by weight, a polycaprolactone oligomer was formulated with 100 parts by weight of a cellulose acetate resin having a molecular weight of approximately 150,000 and acetylated degree of 51%, followed by kneading with a twin screw melting extruder to prepare pellets.

Further, color plates for evaluation of physical properties were molded from the pellets using an injection machine. As evaluation tests, there were conducted a tensile test, a flexural test, an Izod impact strength test, a heat distortion temperature test, and a color hue test. Table 1-4 shows results. Further, a bleeding test was likewise conducted using the color plates. Table 1-5 shows results.

**Table 1-4**

| Evaluation items | | Unit | Example 1-8 | Comparative Example 1-3 |
|---|---|---|---|---|
| Tensile test | strength at yield point | kgf/cm² | absence | 470 |
| | elongation at yield point | % | absence | 16.1 |
| | strength at break | kgf/cm² | 780 | 490 |
| | elongation at break | % | 21.8 | 29.1 |
| | tensile elasticity | kgf/cm² | 11600 | 7900 |
| Flexural test | flexural strength | kgf/cm² | 960 | 560 |
| | flexural elasticity | kgf/cm² | 26700 | 16900 |
| Izod impact test | | kgf.cm/cm² | 3.4 | 10.6 |
| Heat distorsion temperature | high load | °C | 68.1 | 69.4 |
| | low load | °C | 85.0 | 88.9 |
| Color hue | L | % | 87.0 | 79.8 |
| | a | % | -3.8 | -2.6 |
| | b | % | 25.6 | 28.0 |
| | Y1 | % | 49.8 | 60.8 |

**Table 1-5**

| | | unit | 0 hour | 60 hours | 100 hours |
|---|---|---|---|---|---|
| Examples 1-8 | In a hot air circulating dryer at 80°C | % by weight | 100 | 99.9 | 99.9 |
| | In an apparatus maintained at constant temperature and humidity at 50°C, 80%RH | % by weight | 100 | 105.8 | - |
| Comparative Examples 1-3 | In a hot air circulating dryer at 80°C | % by weight | 100 | 99.6 | 99.4 |
| | In an apparatus maintained at constant temperature and humidity at 50°C, 80%RH | % by weight | 100 | 104.5 | - |
| Note: It appears that values exceeding 100% by weight depend upon moisture absorption. | | | | | |

It was confirmed that by formulating the citrate compound of the first aspect of the Invention with a cellulose acetate resin, there can be obtained a cellulose acetate resin composition having an excellent plasticity, and the cellulose acetate resin composition shows an excellent tendency in miscibility each other and, moreover, it is more excellent in view of a moving property toward surface of a molded article as a plasticizer and weight retention ratio in the case of blowing hot air compared to, for example, a conventional polycaprolactone polyol which is a publicly-known plasticizer.

### <Example 2-1>

In proportion of 10 parts by weight and 20 parts by weight, respectively, trisethoxy carbonylmethyl citrate obtained by the same preparation method as in the Example 1-2 was formulated with 100 parts by weight of a commercially supplied polyvinylchloride resin, followed by kneading with a small size kneading apparatus (Laboplastomill). Table 2-1 (1) shows an evaluation result of kneading workability during kneading. It is to be noted that trisethoxy carbonylmethyl citrate employed was identified from measurement results by ¹³C-NMR and FT-IR.

### <Example 2-2>

In proportion of 10 parts by weight and 20 parts by weight, respectively, trisethoxy carbonylmethyl acetyl citrate obtained by the same preparation method as in the Example 1-4 was formulated with 100 parts by weight of a commercially supplied polyvinylchloride resin (the same resin as in the Example 2-1, hereinafter, the same), followed by kneading with a small size melting kneading apparatus (Laboplastomill). Table 2-1 (1) shows an evaluation result of kneading workability during kneading.

It is to be noted that trisethoxy carbonylmethyl acetyl citrate employed was identified from measurement results by ¹³C-NMR and FT-IR.

### <Comparative Example 2-1>

In proportion of 10 parts by weight and 20 parts by weight, dibutyl phthalate (DBP) was formulated with 100 parts by weight of a commercially supplied polyvinylchloride resin, followed by kneading with a small size melting kneading apparatus (Laboplastomill). Table 2-1 (2) shows an evaluation result of kneading workability during kneading.

### <Comparative Example 2-2>

In proportion of 10 parts by weight and 20 parts by weight, tricresyl phosphate (TCP) was formulated with 100 parts by weight of a commercially supplied polyvinylchloride resin, followed by kneading with a small size melting kneading apparatus (Laboplastomill). Table 2-1 (2) shows an evaluation result of kneading workability during kneading.

### <Example 2-3>

A mixture prepared by the same procedures as in the Example 2-1 was kneaded by a twin screw kneading extruder to prepare pellets. Test pieces for physical properties were molded from the pellets by an injection machine to evaluate basic physical properties. Table 2-2 (1) shows results.

### <Example 2-4>

A mixture prepared by the same procedures as in the Example 2-2 was kneaded by a twin screw kneading extruder to prepare pellets. Test pieces for physical properties were molded from the pellets by an injection machine to evaluate basic physical properties. Table 2-2 (1) shows results.

### <Comparative Example 2-3>

A mixture prepared by the same procedures as in the Comparative Example 2-1 was kneaded by a twin screw kneading extruder to prepare pellets. Test pieces for physical properties were molded from the pellets by an injection machine to evaluate basic physical properties. Table 2-2 (2) shows results.

### <Comparative Example 2-4>

A mixture prepared by the same procedures as in the Comparative Example 2-2 was kneaded by a twin screw kneading extruder to prepare pellets. Test pieces for physical properties were molded from the pellets by an injection machine to evaluate basic physical properties. Table 2-2 (2) shows results.

**Table 2-1 (1)**

| | Example 2-1 | | Example 2-2 | |
|---|---|---|---|---|
| PVC/Plasticizer | 100/10 | 100/20 | 100/10 | 100/20 |
| Torque | small | small | small | small |
| Odor | exceedingly small | exceedingly small | small | small |
| Smoking | exceedingly small | exceedingly small | exceedingly small | exceedingly small |
| Leakage of liquid | absence | absence | absence | absence |
| Interlocking | ⓞ | ⓞ | ⓞ | ⓞ |
| Discoloration Plasticizing effect | slightly brown ○ | slightly brown ○ | slightly brown ○ | slightly brown ○ |

**Table 2-1 (2)**

| | Example 2-1 | | Example 2-2 | |
|---|---|---|---|---|
| PVC/Plasticizer | 100/10 | 100/20 | 100/10 | 100/20 |
| Torque | middle | middle | middle | middle |
| Odor | middle | middle | middle | middle |
| Smoking | large | large | large | large |
| Leakage of liquid | absence | absence | absence | absence |
| Interlocking | ○ | ○ | ○ | ○ |
| Discoloration | colorless | colorless | colorless | colorless |
| Plasticizing effect | ○ | | | |

Note: In the Table 2-1 (1) and Table 2-1 (2), "PVC" means a polyvinylchloride resin. As plasticizers, trismethoxy carbonylmethyl citrate was employed in the Example 2-1, and trismethoxy carbonylmethyl acetyl citrate was employed in the Example 2-2. DBP and TCP are employed in the Comparative Examples 2-1 and 2-2, respectively.

**Table 2-2 (1)**

| | Unit | Example 2-3 | | Example 2-4 | |
|---|---|---|---|---|---|
| PVC/Plasticizer | | 100/10 | 100/20 | 100/10 | 100/20 |
| Tensile stress in yield point | kgf/cm² | 410 | 380 | 380 | 360 |
| Tensile elongation in yield point | % | 13.0 | 14.2 | 13.4 | 14.3 |
| Tensile stress in fracture point | kgf/cm² | 480 | 460 | 480 | 440 |
| Tensile elongation in fracture point | % | 120 | 340 | 120 | 360 |
| Tensile elasticity | kgf/cm² | 4400 | 3000 | 4200 | 2900 |
| Flexural stress | kgf/cm² | 660 | 630 | 680 | 610 |
| Flexural elasticity | kgf/cm² | 6300 | 4200 | 5400 | 3700 |
| Izod impact strength | kgf· cm/cm² | 12.8 | not tested | 15.2 | not tested |
| Bleeding property Weight retention ratio | % | 100 | 99.2 | 100 | 99.3 |

**Table 2-2 (2)**

| | Unit | Example 2-3 | | Example 2-4 | |
|---|---|---|---|---|---|
| PVC/Plasticizer | | 100/10 | 100/20 | 100/10 | 100/20 |
| Tensile stress in yield point | kgf/cm² | 400 | 420 | 440 | 420 |
| Tensile elongation in yield point | % | 13.1 | 14.5 | 12.8 | 14.4 |
| Tensile stress in fracture point | kgf/cm² | 500 | 480 | 510 | 480 |
| Tensile elongation in fracture point | % | 160 | 400 | 140 | 380 |
| Tensile elasticity | kgf/cm² | 3800 | 2600 | 4000 | 2600 |
| Flexural stress | kgf/cm² | 710 | 640 | 710 | 650 |
| Flexural elasticity | kgf/cm² | 4300 | 2900 | 4800 | 3100 |
| Izod impact strength | kgf·cm/cm² | 17.6 | not tested | 14.6 | nottested |
| Bleeding property Weight retention ratio | % | 95.6 | 92.2 | 100 | 91.4 |

Note: In the Table 2-2 (1) and Table 2-2 (2), "PVC" means a polyvinylchloride resin.

As plasticizers, trismethoxy carbonylmethyl citrate and trismethoxy carbonylmethyl acetyl citrate are employed, respectively.

As described hereinabove, it was confirmed that the citrate compounds are useful as a plasticizer for a polyvinyl chloride-based resin, and it can provide a polyvinyl chloride-based resin composition having an excellent plasticity.

### <Example 2-5>

In proportion of 10 parts by weight, 20 parts by weight, and 40 parts by weight, respectively, trisethoxy carbonylmethyl citrate obtained by the same preparation method as in the Example 1-2 was formulated with 100 parts by weight of a commercially supplied polystyrene (GPS manufactured by Daicel Chemical Industries, Ltd.), followed by kneading with a small size melting kneading apparatus (Laboplastomill). Table 2-3 (1) shows an evaluation result of kneading workability during kneading. Further, in relation to ones having excellent kneadability, pellets were prepared using a twin screw extruder, and then, test pieces were molded from the pellets by an injection machine to evaluate basic physical properties and bleeding properties. Table 2-4 (1) shows an evaluation result of basic physical properties. Table 2-4 (3) shows an evaluation result of bleeding properties. It is to be noted that trisethoxy carbonylmethyl citrate obtained is identified from measurement results by ¹³C-NMR and FT-IR.

### <Example 2-6>

In proportion of 10 parts by weight, 20 parts by weight, and 40 parts by weight, respectively, trisethoxy carbonylmethyl acetyl citrate obtained by the same preparation method as in the Example 1-4 was formulated with 100 parts by weight of a commercially supplied polystyrene (GPS manufactured by Daicel Chemical Industries, Ltd.), followed by kneading with a small size melting kneading apparatus (Laboplastomill).

Table 2-3 (1) shows an evaluation result of kneading workability during kneading. Further, in relation to ones having excellent kneadability, pellets were prepared using a twin screw extruder, and then, test pieces were molded from the pellets by an injection machine to evaluate basic physical properties and bleeding properties. Table 2-4 (1) shows an evaluation result of basic physical properties. Table 2-4 (3) shows an evaluation result of bleeding properties. It is to be noted that trisethoxy carbonylmethyl acetyl citrate obtained is identified from measurement results by ¹³C-NMR and FT-IR.

### <Comparative Example 2-5>

From a commercially supplied polystyrene (GPS manufactured by Daicel Chemical Industries, Ltd.), samples for an evaluation of basic physical properties and samples for an evaluation of bleeding out were molded using an injection molding machine.

Table 2-4 (2) shows an evaluation result of the basic physical properties, and Table 2-4 (4) shows an evaluation of bleeding out.

### <Comparative Example 2-6>

In proportion of 10 parts by weight, 20 parts by weight, and 40 parts by weight, respectively, dibutyl phthalate (DBP) which is a phthalate-based plasticizer was formulated with 100 parts by weight of a commercially supplied polystyrene (GPS manufactured by Daicel Chemical Industries, Ltd.), followed by kneading with the same small size melting kneading apparatus (Laboplastomill) as one employed in the Examples.

Table 2-3 (2) shows an evaluation result of kneading workability during kneading. Samples having a good kneadability were pelletized in the same formulation using a twin-screw extruder.

Subsequently, in relation to ones having excellent kneadability, pellets were prepared using a twin screw extruder, and then, test pieces were molded from the pellets by an injection machine to evaluate basic physical properties and bleeding properties. Table 2-4 (2) shows an evaluation result of basic physical properties. Table 2-4 (4) shows an evaluation result of bleeding properties.

### <Comparative Example 2-7>

In proportion of 10 parts by weight, 20 parts by weight, and 40 parts by weight, respectively, tricresyl phosphate (TCP) which is a phosphate-based plasticizer was formulated with 100 parts by weight of a commercially supplied polystyrene (GPS manufactured by Daicel Chemical Industries, Ltd.), followed by kneading with the same small size melting kneading apparatus (Laboplastomill) as one employed in the Examples.

Table 2-3 (2) shows an evaluation result of kneading workability during kneading. Samples having a good kneadability were pelletized in the same formulation using a twin-screw extruder.

Subsequently, in relation to ones having excellent kneadability, pellets were prepared using a twin screw extruder, and then, test pieces were molded from the pellets by an injection machine to evaluate basic physical properties and bleeding properties. Table 2-4 (2) shows an evaluation result of basic physical properties, and Table 2-4 (4) shows an evaluation result of bleeding properties.

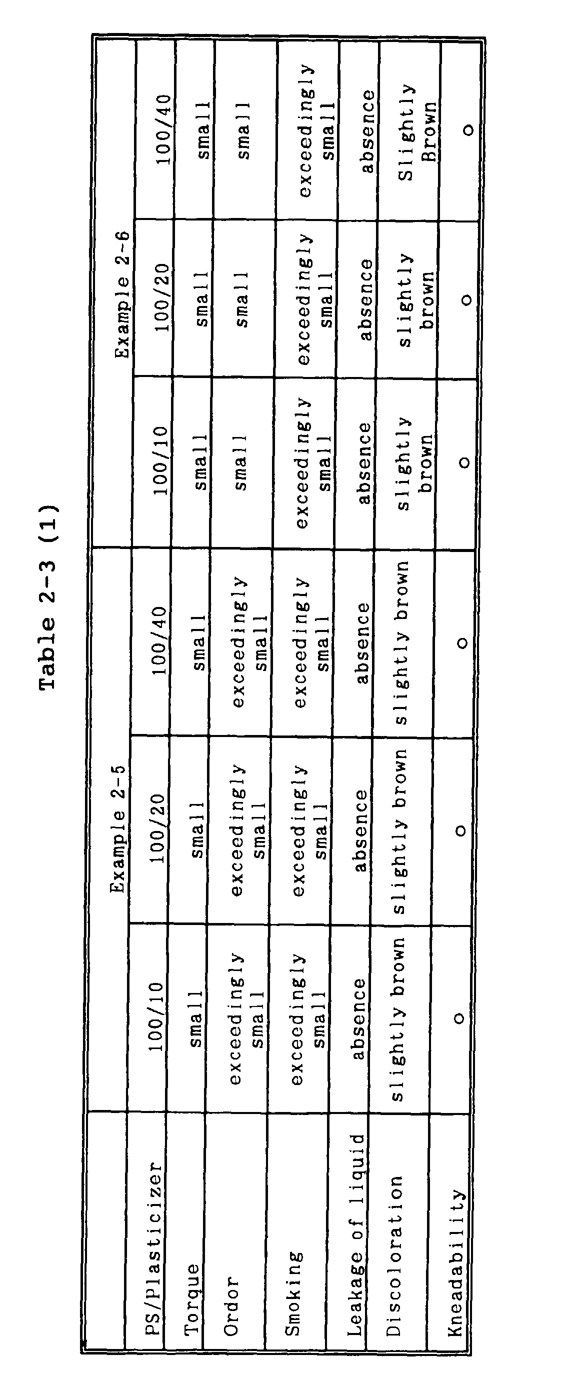

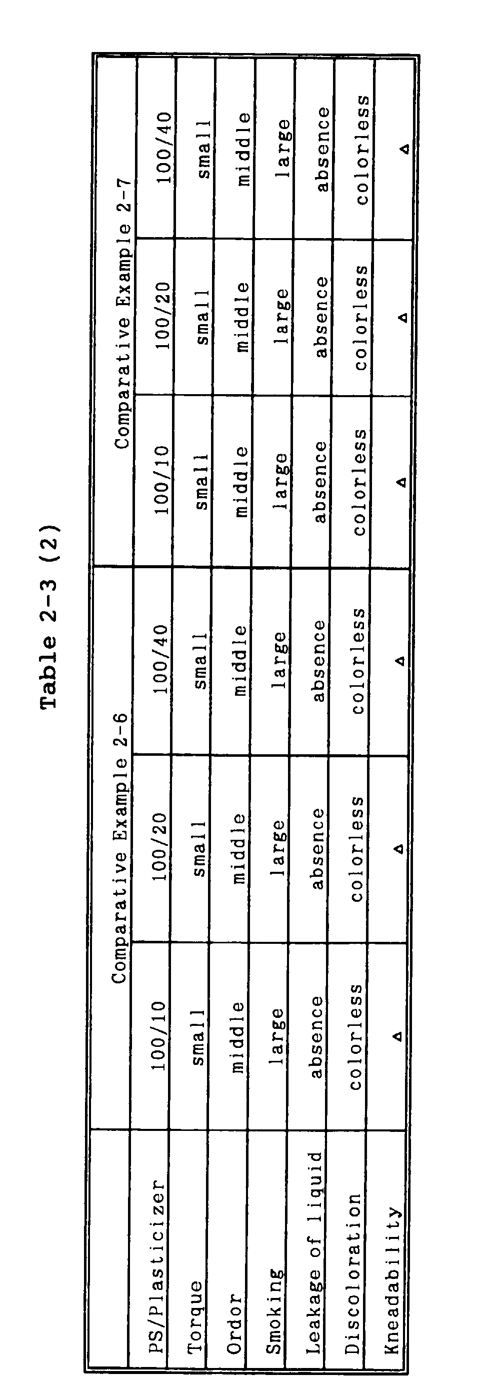

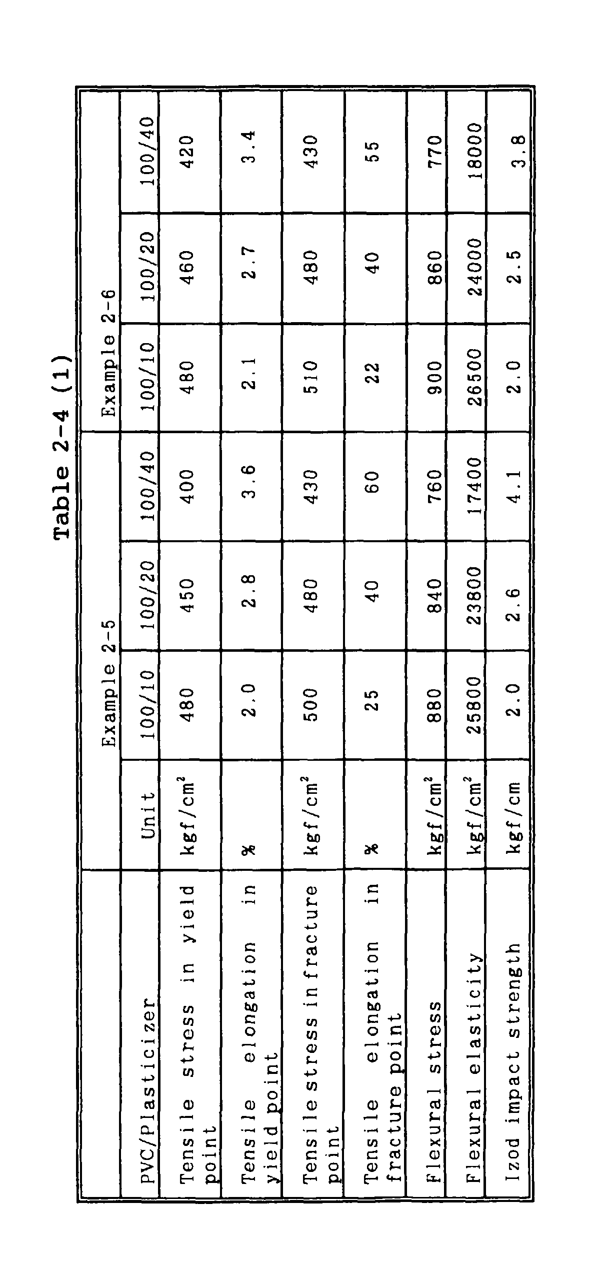

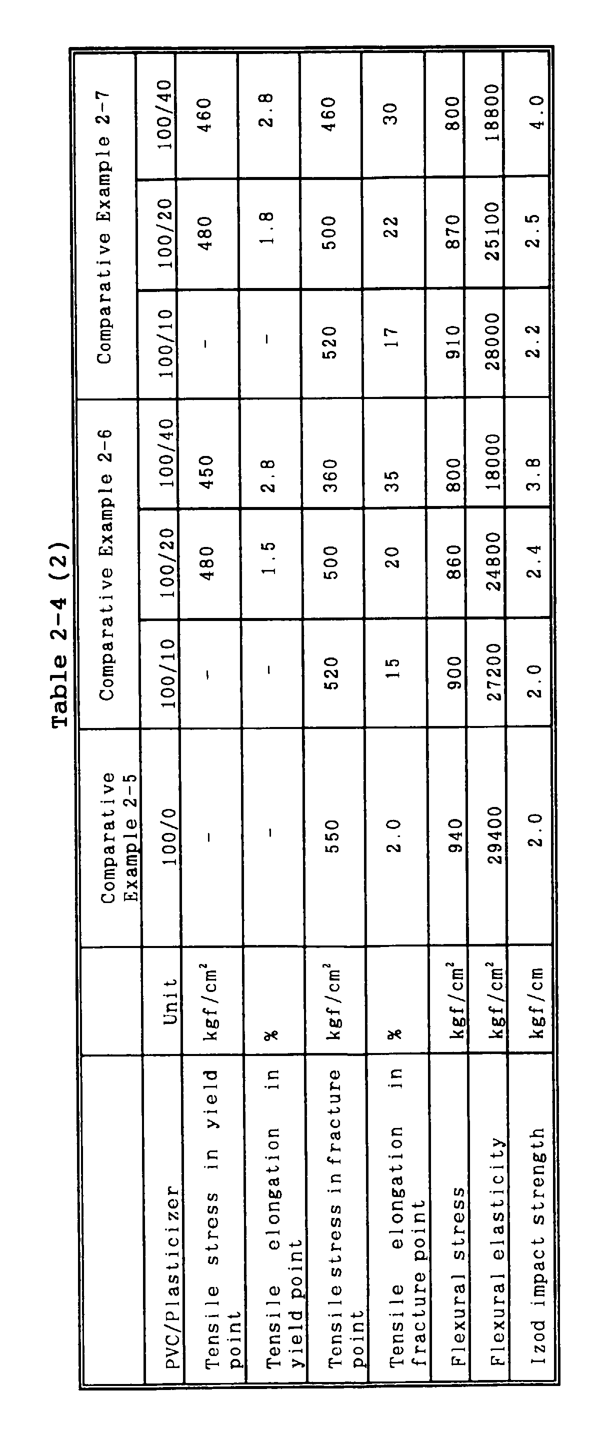

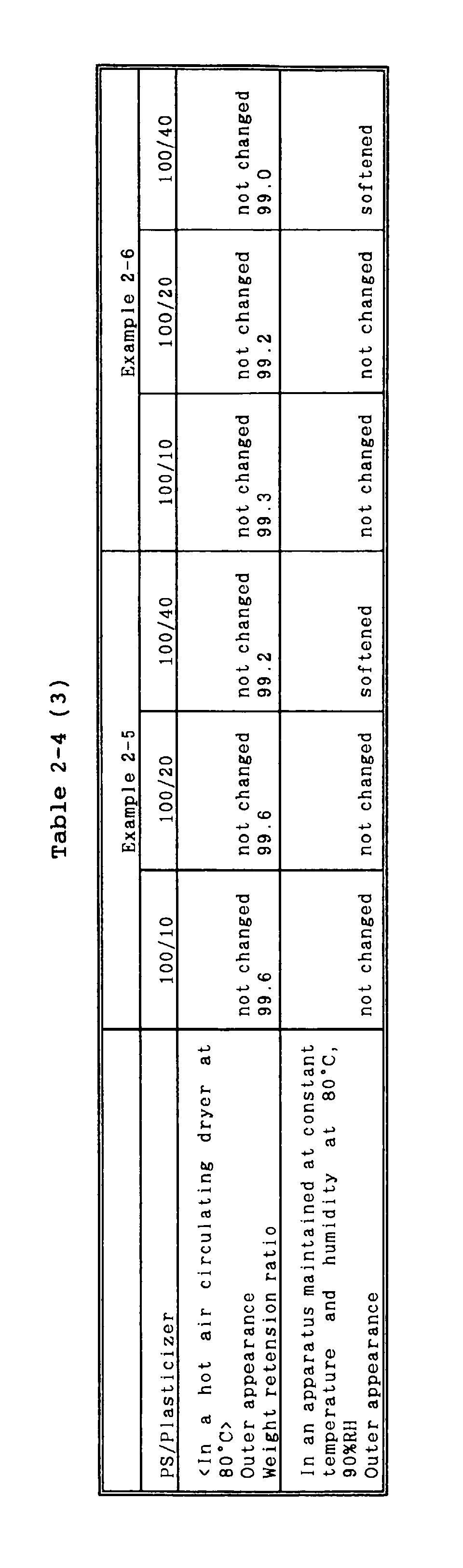

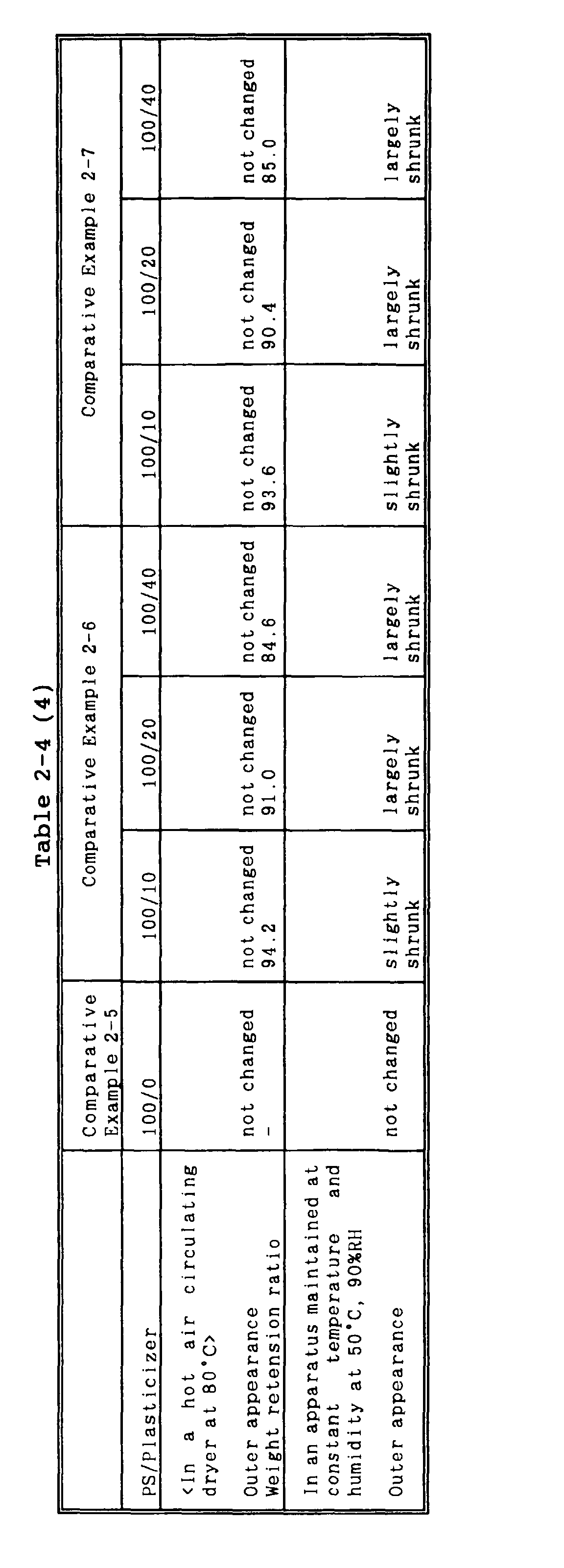

As described hereinabove, the citrate compound shown by the general formula [1] is useful as a plasticizer for a styrene-based resin, and it was confirmed that even in the case that there is prepared a resin composition in which it is formulated in the resin, the citrate compound can almost solve a variety of problems accompanied by the addition of conventional plasticizers.

In Examples and Comparative Examples described below, a cellulose acetate employed has a molecular weight of 150,000 and an acetyl degree of 51%.

Tensile test was conducted according to JIS K7113.

Further, samples in respective tests were supplied in tests after moisture-controlling at 23° and 50% RH in a thermostatic and moisture-controllable apparatus for 24 hours.

Biodegradability test was conducted according to JIS K6950 by a simple experimentation for degradability using a standard active sludge.

### <Example 2-7>

In proportion of 20 parts by weight, 40 parts by weight, and 100 parts by weight, respectively, trisethoxy carbonylmethyl citrate was formulated with 100 parts by weight of a cellulose acetate, and pellets were prepared using a twin screw extruder. From the pellets, a sheet having thickness of 0.5 mm was prepared using a single screw kneading extruder equipped with a T-die.

### <Example 2-8>

A sheet was likewise prepared as in the Example 2-7 except that trisethoxy carbonylmethyl acetyl citrate was employed as a plasticizer.

### <Comparative Example 2-7>

A sheet was likewise prepared as in the Example 2-7 except that a polycaprolactone oligomer PCL305 (a number average molecular weight of approximately 500 manufactured by Daicel Chemical Industries, Ltd.) was employed as a plasticizer.

### <Comparative Example 2-8>

A sheet was likewise prepared as in the Example 2-7 except that DEP (diethyl phthalate) was employed as a plasticizer.

### [Tensile test]

Sheets were cut into a rectangular piece (a longitudinal direction is MD direction (machine direction)) of the width of 10 mm, which were prepared in the Examples 2-7 and 2-8 and the Comparative Examples 2-7 and 2-8. Those were employed for evaluation of mechanical properties (tensile test). Table 2-5 shows results.

### [Weight retention ratio after thermally drying]

Sheets were cut into the square piece of 50 mm, which were prepared in the Examples 2-7 and 2-8 and the Comparative Examples 2-7 and 2-8. Those were weighed, and placed in a hot air recirculating dryer (temperature of 80°C) for a fixed time of period, and an appearance in bleeding out of a plasticizer was observed. At the same time, the weight was measured after wiping the surface of the sheets with a gauze, and the weight retention ratio was measured by comparing to the weight before thermally drying by hot air, and shown by percentage. Table 2-6 shows results.

As a result, it was confirmed that the weight retention ratio after thermally drying (at 80°C in a hot air recirculating dryer for 24 hours) is 99% by weight in a cellulose acetate-based resin-made card containing 40 parts by weight of the plasticizer of the present invention based on 100 parts by weight of the cellulose acetate.

### [Biodegradability]

Sheets obtained in the Examples 2-7 and 2-8 and the Comparative Examples 2-7 and 2-8 were crushed and powdered in a freezing crusher, followed by conducting an evaluation of biodegradability. Table 2-7 shows results.

### [Printing applicability and storage stability]

Cyan, Magenta, yellow, and black inks were gravure-printed on the sheets obtained in the Examples 2-7 and 2-8, and printing was clearly conducted. Further, printed sheets were likewise thermally-dried as in the above-described test of the weight retention ratio after thermally drying, and abnormal portion was not observed in the printed surface.

On the other hand, printing was likewise conducted on the sheets immediately after molding obtained in the Comparative Examples 2-7 and 2-8. As a result, adhering of inks was worse on surface of the test piece in the Comparative Example 2-7 because of the plasticizer bled out of the surface. In the test piece of the Comparative Example 2-8, printing was possible. Printed sheets obtained were likewise thermally as in the above-described tests for the weight retention ratio and, as a result, an unidentifiable blotting was measured on the printed surface in the Comparative Example 2-7 and, floatation and blotting of inks by volatilization of the plasticizer were caused in the Comparative Example 2-8.

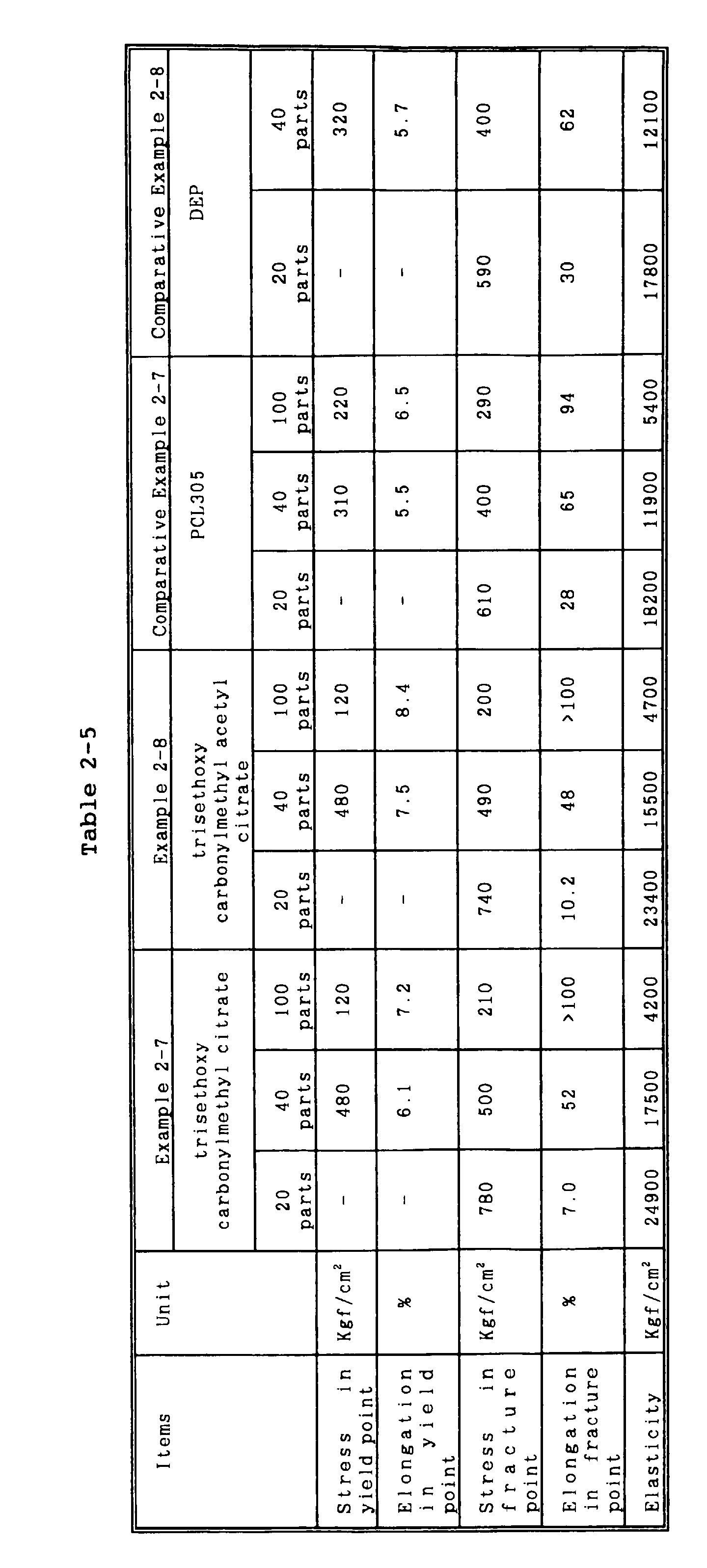

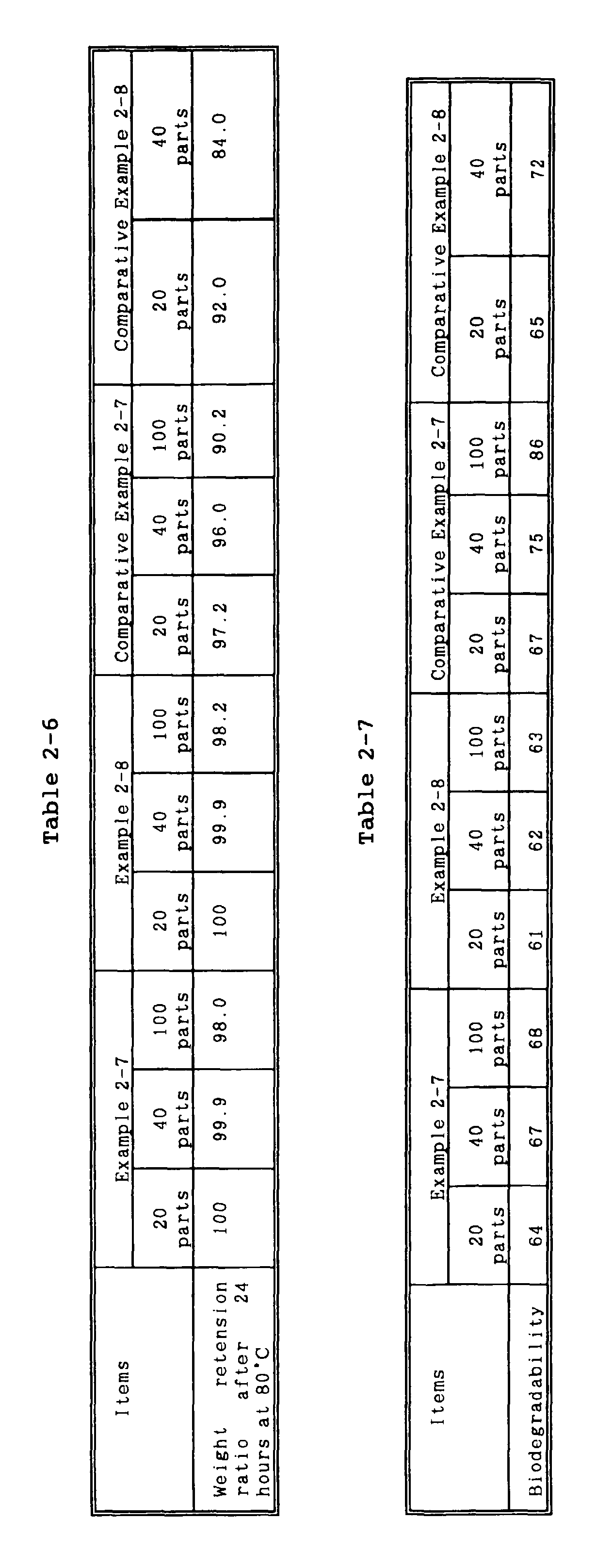

According to the present invention, it was able to obtain a card which is excellent in printing applicability, and which is biodegradable during dumping after use and, in which physical properties are not different from those of general-purpose resins by a cellulose acetate based-resin and a specified citrate compound.

While the invention has been described in detail and with reference to specific embodiments thereof, it will be apparent to one skilled in the art that various changes and modifications can be made therein without departing from the spirit and scope thereof.

## Claims

1. A citrate compound represented by general formula [1] described below, 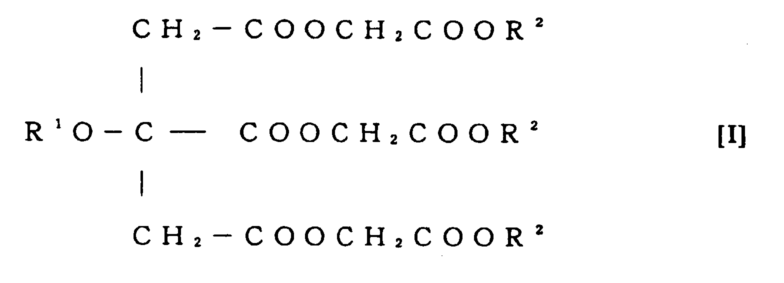 <in the formula, R¹ is a hydrogen atom or an aliphatic acyl group, and R² is an alkyl group>.

2. A citrate compound as claimed in claim 1, wherein said R¹ is a hydrogen atom or an aliphatic acyl group having a carbon number of 1-5.

3. A citrate compound as claimed in claim 1 or 2, wherein said R² is an alkyl group having a carbon number of 1-4.

4. A citrate compound as claimed in claim 1, wherein said R¹ is a hydrogen atom and said R² is a methyl group or an ethyl group.

5. A citrate compound as claimed in claim 1, wherein said R¹ is an acetyl group and said R² is a methyl group or an ethyl group.

6. A plasticizer for a thermoplastic resin which comprises a citrate compound as claimed in any one of claims 1-5.

7. A plasticizer for a thermoplastic resin as claimed in claim 6, wherein said thermoplastic resin is an cellulose acetate-based resin.

8. A plasticizer for a thermoplastic resin as claimed in claim 6, wherein said thermoplastic resin is a polyvinyl chloride-based resin.

9. A plasticizer for a thermoplastic resin as claimed in claim 6, wherein said thermoplastic resin is a styrene-based resin.

10. A thermoplastic resin composition which comprises a thermoplastic resin and a citrate compound as claimed in any one of claims 1-5.

11. A thermoplastic resin composition which comprises 100 parts by weight of a thermoplastic resin and 5-200 parts by weight of a citrate compound as claimed in claim 10.

12. A thermoplastic resin composition as claimed in claim 10, wherein said thermoplastic resin is a cellulose acetate-based resin.

13. A thermoplastic resin composition as claimed in claim 11, wherein said thermoplastic resin is a cellulose acetate-based resin.

14. A thermoplastic resin composition as claimed in claim 12 or 13, wherein an acetylated degree in said cellulose acetate-based resin is 40.03-62.55%.

15. A thermoplastic resin composition as claimed in claim 10 or 11, wherein said thermoplastic resin is a polyvinyl chloride-based resin.

16. A thermoplastic resin composition as claimed in claim 10 or 11, wherein said thermoplastic resin is a styrene-based resin.

17. A cellulose acetate-based resin-made card which comprises a cellulose ester-based resin and a plasticizer which is not apt to bleed out of a cellulose ester-based resin.

18. A cellulose acetate-based resin-made card as claimed in claim 17, wherein said plasticizer is a citrate compound represented by general formula [2] described below, 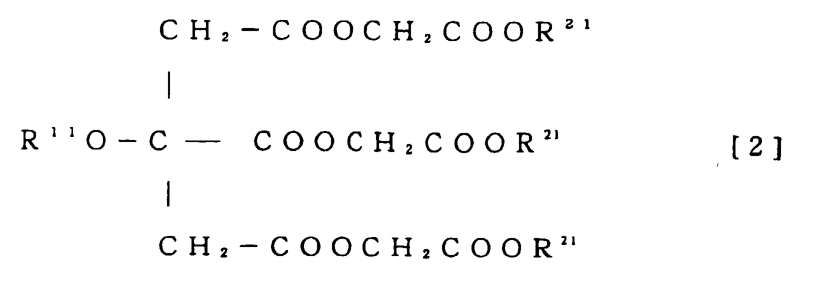 <in the formula, R¹¹ is a hydrogen atom or an aliphatic acyl group having a carbon number of 1-5, and R²¹ is an alkyl group having a carbon number of 1-4>.

19. A cellulose acetate-based resin-made card as claimed in claim 17 or 18, wherein said plasticizer is trisethoxy carbonylmethyl citrate, trisethoxy carbonylmethyl acetyl citrate, or a mixture thereof.

20. A cellulose acetate-based resin-made card as claimed in any one of claims 17-19 which comprises 100 parts by weight of a cellulose acetate-based resin and 5-200 parts by weight of a plasticizer.

21. A cellulose acetate-based resin-made card as claimed in any one of claims 17-20, wherein weight retention ratio after thermally drying (at 80°C for 24 hours in a hot air circulating dryer) is not less than 99% in said cellulose acetate-based resin-made card containing 100 parts by weight of a cellulose acetate-based resin and not more than 40 parts by weight of a plasticizer.
